(19) Europäisches Patentamt
European Patent Office
Office européen des brevets

(11) Veröffentlichungsnummer: **0 209 875**
**B1**

(12) **EUROPÄISCHE PATENTSCHRIFT**

(45) Veröffentlichungstag der Patentschrift: **29.08.90**

(51) Int. Cl.⁵: **C 07 D 265/38,** G 01 N 33/52

(21) Anmeldenummer: **86109957.0**

(22) Anmeldetag: **21.07.86**

# (54) Resorufin-Derivate sowie Verfahren zu deren Herstellung.

(30) Priorität: **25.07.85 DE 3526565**

(43) Veröffentlichungstag der Anmeldung:
**28.01.87 Patentblatt 87/05**

(45) Bekanntmachung des Hinweises auf die Patenterteilung:
**29.08.90 Patentblatt 90/35**

(84) Benannte Vertragsstaaten:
**AT BE CH DE FR GB IT LI LU NL SE**

(56) Entgegenhaltungen:
**DE-A-2 834 743**
**GB-A- 807 687**

(73) Patentinhaber: **BOEHRINGER MANNHEIM GMBH**
**Sandhofer Strasse 116**
**D-6800 Mannheim 31 (DE)**

(72) Erfinder: **Klein, Christian, Dr.**
**Blütenstrasse 16**
**D-8120 Weilheim (DE)**
Erfinder: **Batz, Hans-Georg, Dr. rer. nat.**
**Traubinger Strasse 63**
**D-8132 Tutzing (DE)**
Erfinder: **Herrmann, Rupert, Dr. rer. nat.**
**In der Au 23**
**D-8120 Weilheim (DE)**

**EP 0 209 875 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft Resorufin-Derivate, die als fluoreszenzmarkierte nieder- oder hochmolekulare Verbindungen vielfältige Anwendung finden können, sowie ein Verfahren zu deren Herstellung.

Fluoreszierende Verbindungen finden aufgrund ihrer Fähigkeit, nach Anregung Licht bestimmter Wellenlängen auszusenden, weite Verwendung als markierte Verbindungen in chemischen und biologischen Verfahren, wie z.B. in der klinischen Analytik, aber auch auf immer mehr neuen Gebieten. In der Biochemie finden Fluoreszenzfarbstoffe als hochempfindliche Markierung zunehmende Anwendung. Dabei werden sowohl Verbindungen aus Fluoreszenzfarbstoffen mit niedermolekularen als auch hochmolekularen Substanzen verwendet. Als Beispiele für den breiten Einsatzbereich solcher Fluoreszenzkonjugate seien genannt:

Fluoreszensimmunoassays, für die entweder ein Hapten, Antigen oder eine spezifischer Antikörper mit einem Fluoreszenzfarbstoff markiert eingesetzt wird. Durch die spezifische Bindung des Antikörpers an Hapten oder Antigen kann nach verschiedenen Verfahren die Konzentration dieser Stoffe oder des Antikörpers bestimmt werden.

In der Immunofluoreszenz-Mikroskopie werden Antigene, wie z.B. ganze Zellen oder Proteine durch fluoreszenzmarkierte Antikörper unter dem Mikroskop sichtbar gemacht (Wang et al., Meth. Enzymology *85,* 514 ff).

Ebenso kann die Verteilung eines Haptens oder Antigens in einer Zelle direkt beobachtet werden, wenn die betreffende Verbindung fluoreszenzmarkiert in die Zelle eingebracht und unter dem Mikroskop verfolgt wird.

Fluoreszenzmarkierte Latexpartikel finden Verwendung bei der Sortierung von Zellen im "Fluorescent Activated Cell Sorter" (FACS).

Nicht zuletzt dienen Substrate, die eine fluoreszierende Markierung tragen zur Bestimmung und der Messung der Aktivität von Enzymen.

Kompetitive Immunoassays basieren auf der Konkurrenz zwischen einem in einer Probe zu bestimmenden Liganden und einem markierten, in bekannter Konzentration vorliegenden Liganden um eine bekannte, aber begrenzte Anzahl von Ligandenbindungsstellen auf Antikörpern, welche sowohl für die zu bestimmenden als auch für die markierten Liganden spezifisch sind. Die Konzentration des zu bestimmenden Liganden in der Probe ist ausschlaggebend dafür, wieviel markierte Ligandenmoleküle an die Antikörper gebunden werden. Die Konzentration an Komplexen aus Antikörpern und fluoreszenzmarkierten Liganden kann mit spektroskopischen Methoden ermittelt werden. Sie ist umgekehrt proportional zu der in der Probe befindlichen zu bestimmenden Ligandenkonzentration. Als markierte Liganden, die der Probe mit dem zu bestimmenden Liganden in bekannter Konzentration zugesetzt werden, wurden ursprünglich überwiegend mit Radioisotopen markierte Liganden verwendet. Wegen der bekannten Nachteile einer Radioisotopenmarkierung gewinnt die Markierung mit fluoreszierenden Verbindungen immer mehr an Bedeutung. Fluoreszierende Verbindungen werden hierbei an Moleküle der zu bestimmenden Substanz gebunden. Diese Konjugate können dann prinzipiell für die unterschiedlichsten

Fluoreszenzimmunoassays, wie z.B.
Fluoreszenzpolarisations-Immunoassay,
Fluoreszenz-Quenching-Immunoassay oder
Fluoreszenz-Enhancement-Immunoassay, eingesetzt werden.

Als Markierung eignen sich prinzipiell alle Fluoreszenzfarbstoffe, die einen großen Extinktionskoeffizienten und eine hohe Quantenausbeute sowie ausreichende Stabilität unter den Testbedingungen besitzen. Bisher wurden deshalb überlicherweise Fluorescein oder Fluoresceinderivate verwendet (J. Landon & R. S. Kamel in: Immunoassays 80s, Univ. Park Press, Baltimore, Md., 1980, Seite 91—112). Die mit Fluorescein oder dessen Derivaten markierten hoch- oder niedermolekularen Verbindungen besitzen jedoch Nachteile. Absorptions- und Emissionsmaxima der fluorescein-markierten Substanzen liegen im Wellenlängenbereich zwischen 490 und 520 nm. Da ein erheblicher Teil analytischer Methoden vor allem der Fluoreszenzimmunoassay-Verfahren in Körperflüssigkeiten, wie z.B. Serum, durchgeführt wird, treten in dem genannten Spektralbereich Störungen durch die Eigenfluoreszenz biologischen Materials in den Proben auf. Hauptverantwortlich hierfür ist Bilirubin, das ebenfalls in dem Bereich um 500 nm Licht absorbiert und Fluoreszenz emittiert.

Manche Meßanordnungen erfordern Marker-Substanzen mit einem möglichst großen Stokes-Shift. Bei den Fluoresceinderivaten beträgt dieser Shift maximal 30 nm. Dies führt zu Lichtstreuungsproblemen, welche die Empfindlichkeit der Fluoreszenzmessungen beeinträchtigen. Für solche Fälle wären Verbindungen mit einem größeren Stokes-Shift als dem von Fluoresceinderivaten wünschenswert.

Aufgabe der vorliegenden Anmeldung war es Verbindungen bereitzustellen, die diese Nachteile nicht mehr aufweisen. Gelöst wird diese Aufgabe durch die erfindungsgemäßen Resorufin-Derivate.

Gegenstand der Erfindung sind somit Resorufin-Derivate der allgemeinen Formeln Ia und IB

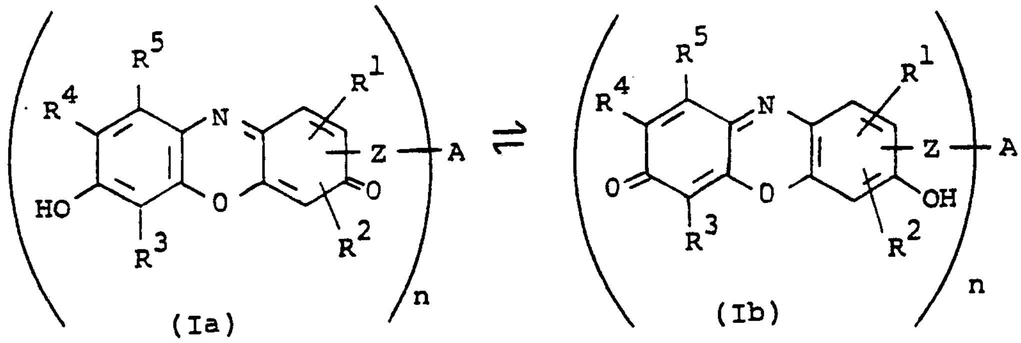

in denen

$R^1$, $R^2$, $R^3$, $R^4$ und $R^5$, die jeweils gleich oder verschieden sein können, Wasserstoff, Halogen, eine Carboxy-, Carboxamido-, Niederalkoxycarbonyl-, Cyano-, Nitrogruppe, einen Niederalkyl- oder Niederalkoxy-Rest, die jeweils durch Carboxy-, Carboxamido-, Niederalkoxycarbonyl-, Cyano- oder Nitrogruppen substituiert sein können, wobei $R^4$ und $R^5$ zusammen einen anellierten Benzol- oder Naphthalinring vorstellen können, der unsubstituiert ist oder einen oder mehrere Substituenten, ausgewählt aus der Gruppe $SO_3H$, $CO_2H$ oder $C_1$—$C_5$-Alkoxy, trägt,

Z ein Brückenglied

A den Rest eines Liganden

n eine ganze Zahl von 1 bis 200 bedeuten.

Die Niederalkyl- bzw. Niederalkoxygruppen in der Definition von $R^1$, $R^2$, $R^3$, $R^4$ und $R^5$ enthalten Kohlenwasserstoffketten mit 1 bis 5, vorzugsweise 1 bis 3 Kohlenstoffatomen. Besonders bevorzugt sind Methyl und Ethyl bzw. Methoxy und Ethoxy.

Halogen in der Definition von $R^1$, $R^2$, $R^3$, $R^4$ und $R^5$ bedeutet Fluor, Chlor, Brom oder Jod. Chlor und Brom sind besonders bevorzugt.

Der von den Substituenten $R^4$ und $R^5$ gegebenenfalls gebildete anellierte Aromat ist vorzugsweise Benzol.

Das Brückenglied Z wird durch übliche Additions- bzw. Kondensationsreaktionen zwischen einem reaktiven Substituenten $X^1$ des Resorufin-Grundgerüstes und einer reaktiven Gruppe $X^2$ des Liganden bzw. Ligandenanalogons, gegebenenfalls unter Zwischenschalten einer bifunktionellen Verbindung $X^3$—M—$X^4$, in der $X^3$ und $X^4$ reaktive Gruppen und M den restlichen Molekülteil darstellen, gebildet.

In der folgenden Tabelle 1 sind beispielhaft einige mögliche Bedeutungen für solche reaktiven Substituenten $X^1$ und $X^2$ sowie die bei der Umsetzung resultierenden Brückenglieder Z aufgeführt.

Tabelle 1: Einige Bedeutungen für die reaktiven Gruppen $X^1$, $X^2$ und das daraus resultierende Brückenglied Z

| $X^1$ | $X^2$ | Z |
|---|---|---|
| -COOH | $-NH_2$ | -CONH- |
| -COOT* | $-NH_2$ | -CONH- |
| $-COOCO_2T^1$** | $-NH_2$ | -CONH- |
| -NCO | $-NH_2$ | -NHCONH- |
| -NCS | $-NH_2$ | -NHCSNH- |
| 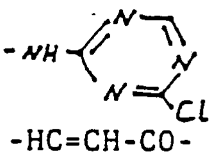 | $-NH_2$ | 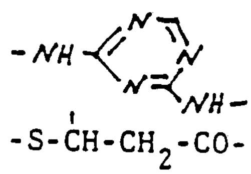 |
| -HC=CH-CO- | -SH | $-S-CH-CH_2-CO-$ |
| -CHO | $-NH_2$ | -CH=N- |
| $-SO_2Cl$ | $-NH_2$ | $-SO_2NH-$ |
| $-COCH_2$-Halogen | -SH | $-COCH_2-S-$ |
| $-COCH_2$-Halogen | -OH | $-COCH_2-O-$ |
| $-(CH_2)_m-NH_2$*** | -COOH | $-(CH_2)_m-NH-CO-$ |
| 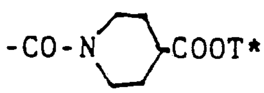 | $-NH_2$ | 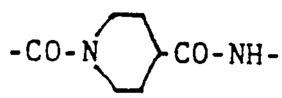 |

* T bedeutet einen Alkylrest mit 1 bis 5 Kohlenstoffatomen oder eine elektronegative aktivierte Estergruppe, wie z. B. die N-Hydroxysuccinimidestergruppe

** $T^1$ bedeutet einen Alkylrest mit 1 bis 5 Kohlenstoffatomen

*** m bedeutet eine ganze Zahl von 0 bis 3

Statt primärer Amine wie in Tabelle 1 aufgeführt, können als reaktive Gruppen $X^1$ oder $X^2$ sinngemäß auch sekundäre Amine unter Ausbildung entsprechender Produkte um Einsatz kommen.

Als bifunktionelle Verbindungen $X^3$—M—$X^4$ sind Diamine, Dicarbonsäuren sowie deren Derivate, Dialdehyde, Aminocarbonsäuren und weitere Verbindungen, die überlicherweise zur Herstellung derartiger Verknüupfungen eingesetzt werden, bevorzugt. Beispielhaft für solche Substanzen werden Piperazin, 1,2-Ethylen-diamin, Bernsteinsäure, Glutardialdehyd, Glycin, Sarcosin, β-Alanin und Piperidin-4-carbonsäure genannt.

Unter einem Liganden in der Definition von A sind Haptene, Antigene, Antikörper, Substrate sowie Träger und hiervon abgeleitete Verbindungen zu verstehen,

Unter einem Hapten ist erfindungsgemäß eine Substanz mit niederem Molekulargewicht zu verstehen, die in der Regel allein nicht in der Lage ist, Antikörper zu erzeugen. Als Substanzen mit niederem Molekulargewicht werden Verbindungen mit einem Molekulargewicht von etwa 100 bis 2000 bezeichnet. Beispiele für solche Substanzen sind physiologisch aktive Substanzen, die im Säugetier oder menschlichen Organismus vorliegen, sowie deren Metabolite oder Arzneimittel, die an Tiere oder Menschen verabreicht werden sowie Metabolite hiervon. Unter dem Begriff Hapten können jedoch auch alle weiteren niedermolekularen Verbindungen verstanden werden, sofern sie nur ein Molekulargewicht in dem oben angegebenen Bereich aufweisen. Beispiele für mögliche Haptene sin Amine, Steroide, Hormone, Kohlenhydrate, Peptide, Oligonukleotide, Kombinationen hiervon.

Antigene sind hochmolekulare Verbindungen. Solche sind Üblicherweise in der Lage, in damit behandelten Organismen — Antikörper zu erzeugen. Hochmolekulare Verbindungen sind erfindungsgemäß solche, die mindestens ein Molekulargewicht von etwa 2000, vorzugsweise jedoch ein solches von mindestens etwa 5000 aufweisen. Das Molekulargewicht solcher Verbindungen kann nach oben nicht beschränkt werden. Es kann Werte bis zu 20 Millionen betragen, es kann aber auch darüber liegen. Antigene, die als Liganden in Verbindungen der allgemeinen Formeln Ia/Ib verkommen können, sind z.B. Proteine, Nukleinsäuren, Polysaccharide, Kombinationen hiervon oder andere hochmolekulare Substanzen. Erfindungsgemäß werden unter dem Begriff Antigen alle hochmolekularen Verbindungen verstanden, die ein Mindestmolekulargewicht von etwa 2000 aufweisen.

Antikörper sind alle jene Proteine oder Glycoproteine, die mit Antigenen oder Haptenen sowie hiervon abgeleiteten Verbindungen spezifisch reagieren und einen Komplex bilden. Erfindungsgemäß können als Ligand in Verbindungen der allgemeinen Formel Ia/Ib intakte Antikörper oder auch Fragmente hiervon eingesetzt werden. Auch diese Fragmente können erfindungsgemäß als Antikörper bezeichnet werden, solange sie in der Lage sind, Antigene, Haptene sowie hiervon abgeleitete Verbindungen zu binden.

Unter Substraten werden Verbindungen verstanden, die in einer chemischen Reaktion eine nachweisbare Veränderung erfahren. Beispielsweise können hierunter alle diejenigen Verbindungen oder hiervon abgeleiteten Stoffe verstanden werden, auf die Enzyme einwirken, wie z.B. Aminosäuren, Peptide, Proteine, Glukoside, Oligo- oder Polysaccharide, Nukleotide, Nukleinsäuren, Kombinationen hiervon oder andere enzymatisch veränderbare Substanzen.

Träger können natürlich vorkommende oder synthetische, vernetzte oder nicht-vernetzte Materialien mit oder ohne bestimmte Form sein. Es sind hierunter einzelne Verbindungen oder Mischungen von Verbindungen zu verstehen. Als Träger können z.B. Verbindungen oder Mischungen von Verbindungen, wie Polysaccharide, Nukleinsäuren, Peptide, Proteine, Kombinationen hiervon oder auch Gummi, Lignin, Glas, Kohle, synthetische Additions- oder Kondensationspolymere, wie Polystyrol, Polyacryl, Vinylverbindungen, Polyester, Polyether und Polyamide oder auch komplexe Gebilde, wie Latexpartikel, Vesikel, Liposomen, Zellwandteile oder sogar ganze Zellen Verwendung finden.

Eine von einem bestimmten Liganden abgeleitete Verbindung wird als Ligandenanalogon bezeichnet. Unter einem Ligandenanalogon wird eine Substanz verstanden, die sich strukturell geringfügig von dem entsprechenden Liganden unterscheidet, hinsichtlich ihrer Eigenschaften jedoch keinen wesentlichen Unterschied aufwest. Der Unterschied kann z.B. auf einem zusätzlichen Substituenten oder einem fehlenden Molekülteil beruhen.

Grundsätzlich können alle Liganden zur Bildung erfindungsgemäßer Verbindungen Ia oder Ib verwendet werden, die freie Amino-, Hydroxyl-, Sulfhydryl- oder Carboxylgruppen tragen, über die sich erstere mit dem Resorufingrundkörper gegebenenfalls unter Zwischenschaltung eines Brückengliedes verknüpfen lassen. Besonders vorteilhaft sind freie Amino- oder Carboxylgruppen. Unter freien Aminogruppen sind sowohl primäre als auch sekundäre Aminogruppen zu verstehen. Besitzen Liganden keine geeigneten Gruppen, so müssen auf synthetischem Wege solche Gruppen, wie z.B. Amino- oder Carboxygruppen, eingeführt werden. Es ist ferner möglich, gegebenenfalls vorhandene, nicht reaktive funktionelle Gruppen von solchen Liganden chemisch zu aktivieren. Da die so entstandenen Substanzen nicht mehr völlig mit den ursprünglichen Verbindungen identisch sind, spricht man von Ligandenanaloga.

Die Zahl n gibt an, wieviele Resorufinmoleküle an einen Liganden oder ein Ligandenanalogon gebunden sind. Diese Zahl hängt ab von der Anzahl reaktiver Gruppen in dem entsprechenden Liganden oder Ligandenanalogon. Je mehr reaktive Gruppen der Ligand oder das Ligandenanalogon besitzen, desto mehr Resorufinmoleküle können gebunden werden. Die Zahl n liegt überlicherweise bei 1 bis 200, besonders bevorzugt bei 1 bis 100.

Ein weiterer Gegenstand der Erfindung ist ein Verfahren zur Herstellung der erfindungsgemäßen Verbindungen der allgemeinen Formeln Ia und Ib. Dieses Verfahren ist dadurch gekennzeichnet, daß man in an sich bekannter Weise Verbindungen der allgemeinen Formeln IIa und IIb

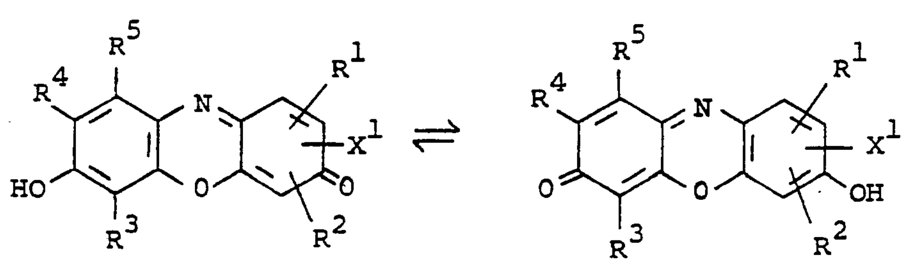

(IIa) (IIb)

in denen

$R^1$, $R^2$, $R^3$, $R^4$ und $R^5$ die in den allgemeinen Formeln Ia und Ib angegebenen Bedeutungen haben und

$X^1$ eine reaktive Gruppe vorstellt,

a) mit einem Liganden der allgemeinen Formel

$$X^2\text{—}A \quad (III)$$

in der

$X^2$ eine reaktive Gruppe und

A den Rest des Liganden vorstellt, oder

b) mit einer bifunktionellen Verbindung der allgemeinen Formel

$$X^3—M—X^4 \quad (IV)$$

in der

$X^3$ und $X^4$ reaktive Gruppen und

M den Rest der bifunktionellen Verbindung bedeuten,

und einem Liganden der allgemeinen Formel

$$X^2—A \quad (III)$$

in der

$X^2$ und A die oben angegebene Bedeutung haben,

umsetzt, wobei gegebenenfalls bei einzelnen Verfahrensschritten Schutzgruppen eingeführt und nachträglich wieder abgespalten sowie einzelne reaktive Gruppen $X^1$, $X^2$, $X^3$ und $X^4$ in andere reaktive Gruppen überführt werden können.

Unter reaktiven Gruppen in der Definition von $X^1$, $X^2$, $X^3$ und $X^4$ können prinzipiell alle üblichen reaktionsfähigen funktionellen Gruppen verstanden werden. Besonders bevorzugt als funktionelle Gruppen sind Säurereste, wie Carbon- oder Sulfonsäure, sowie dabon abgeleitete Gruppen, wie Ester, Amide, Anhydride, Säurehalogenide oder Reste, wie primäres oder sekundäres Amin, Cyanat-, Isocyanat-, Thiocyanat-, Isothiocyanat-, Aldehyd-, Sulfhydryl-, Hydroxy-, α-Ketohalogenidrest. Beispiele für reaktive Gruppen sind in Tabelle 1 unter $X^1$ und $X^2$ exemplarisch genannt. Diese Reste sind selbstverständlich untereinander auszutauschen und können auch die Bedeutung der reaktiven Gruppen $X^3$ und $X^4$ annehmen.

Unter dem Rest M der bifunktionellen Verbindung IV kann prinzipiell jeder organische oder anorganische Rest verstanden werden. Bevorzugt sind jedoch solche Reste, bei denen M einen geradkettigen oder verzweigten aliphatischen, cycloaliphatischen oder aromatischen Rest bedeutet oder eine Kombination solcher Reste darstellt. Ganz besonders bevorzugt sind aliphatische Reste aus 1 bis 10, vorzugsweise 1 bis 7 Kohlenstoffatomen oder solche Reste, die beider oder nur eine der zwei reaktiven Gruppen $X^3$/$X^4$ in einen cycloaliphatischen Rest miteinbeziehen.

Verbindungen der allgemeinen Formeln IIa und IIb erhält man in vorteilhafter Weise aus Nitrosoresorcinderivaten der allgemeinen Formel V

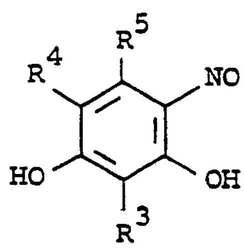

(V)

in der $R^3$, $R^4$ und $R^5$ die in den allgemeinen Formel Ia und Ib angegebene Bedeutung besitzen, mit Resorcin-Derivaten der allgemeinen Formel IV

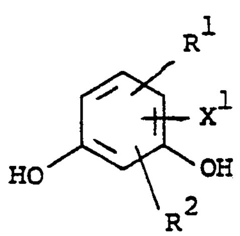

(VI)

in der $R^1$ und $R^2$ die in den allgemeinen Formeln Ia und Ib angegebene Bedeutung besitzen und $X^1$ eine reaktive Gruppe vorstellt.

Die Umsetzung von Verbindungen der allgemeinen Formel V mit Derivaten der allgemeinen Formel VI erfolgt zweckmäßigerweise in Anwesenheit von Braunstein und Schwefelsäure bei niedrigen Temperaturen. Es entstehen dabei zunächst Resazurin-Derivate, die sich leicht in Resorufin-Derivate der allgemeinen Formeln IIa un IIb überführen lassen.

Die Reaktion der Verbindungen der allgemeinen Formel V mit Verbindungen der allgemeinen Formel VI wird überlicherweise bei einer Temperatur zwischen −10 bis 50°C, vorzugsweise zwischen 0 und 30°C durchgeführt. Besonders schonend verläuft die Umsetzung wenn man die Substanzen der allgemeinen Formel V und der Formel VI bei ungefähr 0°C vermischt und das Reaktionsgemisch sich anschließend auf Raumtemperatur erwärmen läßt. Die Konzentration an Braunstein soll zweckmäßigerweise bei 0,5 bis 5,

vorzugsweise bei 1 bis 2 mol/l liegen. Die Schwefelsäurekonzentration sollte 0,5 bis, vorzugsweise 1 bis 3 mol/l betragen.

Die Reduktion der zunächst entstehenden Resazurin-Derivate zu Resorufinen der allgemeinen Formeln IIa und IIb wird vorzugsweise in ammoniakalischer Lösung mit Zinkstaub durchgeführt (vgl. Nietzki et al., Ber. Dtsch. Chem. Ges. *22,* 3020 [1889]) oder mit Natriumborhydrid. Als Lösungsmittel verwendet man zweckmäßigerweise Wasser-Alkohol-Gemische, bevorzugt ein Gemisch aus einem Teil Wasser mit 0 bis 4 Teilen Methanol. Pro Mol zu reduzierender Substanz werden 1 bis 20, vorzugsweise 1 bis 5 Mol Zinkstaub bzw. Natriumborhydrid zugegeben. Die Temperatur der Reaktionslösung wird dabei −10 bis +35°C, vorzugsweise +5 bis +10°C gehalten. Die genaue Einhaltung des Temperaturbereiches hat sich als notwendig für einen eindeutigen Reaktionsverlauf erwiesen. Ohne Kühlung führt die exotherme Reaktion zu Nebenprodukten, die schwer abtrennbar sind.

Unter den gewählten milden Bedingungen verläuft die Umsetzung zwischen den Substanzen der allgemeinen Formel V und der allgemeinen Formel VI eindeutig und mit guter Ausbeute. Der gewählte Syntheseweg ist variationsfähig. Dies eröffnet insbesondere im Hindblick auf die Darstellung unsymmetrisch substituierter Resorufin-Derivate zahlreiche Synthesemöglichkeiten. Bedingt durch dieses äußerst variationsfähige Herstellungsverfahren ist eine Vielzahl von Resorufin-markierten Verbindungen zugänglich, die durch ihre unterschiedlichen Substituenten an unterschiedlichen Positionen im Chromophor einen weiten Farbbereich abdecken.

Vor der Umsetzung der Resorufin-Derivate der allgemeinen Formeln IIa und IIb mit Verbindungen der allgemeinen Formel III bzw. mit Verbindungen der allgemeinen Formeln IV und III werden erstere zweckmäßigerweise in Triacyldihydroresorufin-Derivate der allgemeinen Formeln VII überführt,

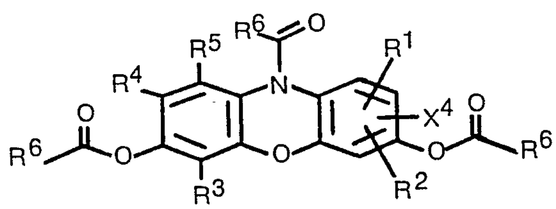

(VII)

in der

$R^1$, $R^2$, $R^3$, $R^4$, $R^5$ und $X^1$ die in den allgemeinen Formeln IIa und IIb angegebene Bedeutung haben und $R^6$ ein Niederalkyl-, Aryl- oder Aralkylrest ist.

Niederalkyl in der Definition von $R^6$ bedeutet eine Alkylgruppe mit 1 bis 5, vorzugsweise 1 bis 3 Kohlenstoffatomen. Besonders bevorzugt sind Methyl und Ethyl. Als Arylgruppe ist der Phenylrest besonders bevorzugt. Der Aralkylrest enthält vorzugsweise als Arylteil eine Phenylgruppe, der Alkylteil enthält 1 bis 5 vorzugsweise 1 bis 3 Kohlenstoffatome. Als Aralkylgruppe ist der Benzylrest ganz besonders bevorzugt.

Zur Herstellung der Triacylderivate der allgemeinen Formel VII werden die entsprechenden Resorufin-Derivate der allgemeinen Formeln IIa und IIb zunächst mit einem starken Reduktionsmittel, wie z.B. Zinn-II-Chlorid oder Chrom-II-acetat, oder elektrochemisch reduziert. Zur Reduktion erwärmt man das Resorufin-Derivat 10 Minuten bis eine Stunde mit 2 bis 10, bevorzugt 2 bis 6 Äquivalenten Reduktionsmittel in einem geeigneten Lösungsmittel, vorzugsweise Zinn-II-chlorid in 5 bis 35 %iger wäßriger Salzsäure. Beim Abkühlen fällt die Dihydroverbindung aus. Die Acylierung erfolgt in üblicher Weise mit einem geeigneten Acylierungsmittel, z.B. Acetanhydrid oder Benzoylchlorid. Bevorzugt werden die Verbindungen der allgemeinen Formel VII in einem Eintopfverfahren durch reduktive Acylierung der Resorufin-Derivate IIa und IIb hergestellt. Das entsprechende Resorufin-Derivat wird hierzu mit 2 bis 6 Äquivalenten-Reduktionsmittel 5 Minuten bis 3 Stunden in Gegenwart des Acylierungsmittel in einem geeigneten Lösungsmittel unter Rückfluß erhitzt oder bei Raumtemperatur 4 bis 16 Stunden gerührt.

In den so erhaltenen Triacyl-dihydroresorufin-Derivaten VII kann die reaktive Gruppe $X^1$ vor der weiteren. Umsetzung gegebenenfalls in eine andere reaktive Gruppe überführt werden. Insbesondere, wenn $X^1$ eine Carboxylgruppe bedeutet, ist es zweckmäßig, diese in eine Carbonsäurechlorid-, Carbonsäureanhydrid oder reaktive Esterfunktion umzuwandeln. Dies kann auf die unterschiedlichste Art und Weise erfolgen, so z.B. mit Carbodiimiden, Alkoholen, Halogeniden oder N-Hydroxysuccinimid. Es stehen dem Fachmann zahlreiche literaturbekannte Verfahren zur Verfügung. Besonders bevorzugt ist die Überführung der Carbonsäurefunktion in ein Carbonsäurechlorid, z.B. mit Thionylchlorid/Dimethylformamid oder Oxalylchlorid/Dimethylformamid, sowie in einen aktivieren Ester, z.B. den N-Hydroxysuccinimidester.

Besitzen Liganden oder Ligandenanaloga freie Amino-, Hydroxyl- oder Sulfhydrylgruppen, so können diese als reaktive Gruppen $X^2$ reagieren. Die Liganden bzw. Ligandenanaloga $X^2$—A können dann direkt mit Verbindungen IIa und IIb, gegebenenfalls nach vorheriger Umwandlung in Triacyl-dihydroresorufin-Derivate oder/und nach Aktivierung der Gruppe $X^1$ unter Bildung von Amid-, Ester oder Thioesterbindungen umgesetzt werden. Aufgrund ihrer Stabilität ist die Bildung mindestens einer Amidbindung besonders bevorzugt, wobei zu deren Bildung vorzugsweise von Verbindungen der allgemeinen Formeln IIa/IIb bzw. VII ausgegangen wird, in denen $X^1$ ein Carbonsäurehalogenid darstellt. Dessen Umsetzung mit einem aminogruppenhaltigen Liganden bzw. Ligandenanalogon erfolgt nach üblichen Methoden, beispielsweise in einem organischen Lösungsmittel, wie Dichlormethan, unter Zusatz eines tertiären

Amins, wie Triethylamin, als Base. Je nach Größe des Liganden bzw. Ligandenanalogons und damit zusammenhängend der Zahl seiner freien Aminogruppen und der eingesetzten Menge an Verbindungen II a/II b können pro Liganden- bzw. Ligandenanalogonmolekül mehrere Chromophore gebunden werden.

Werden zur Herstellung der erfindungsgemäßen Verbindungen der allgemeinen Formeln Ia und Ib Triacyl-Derivate der allgemeinen Formel VII eingesetzt, so müssen nach der Umsetzung die als Schutzgruppen verwendeten Acylreste des Dihydroresorufinteils selektiv abgespalten und der resultierende Leukofarbstoffrest zum Chromophor der Verbindung Ia/Ib oxidiert werden.

Die O-Acylreste des Dihydroresorufinteils werden besonders vorteilhaft durch Reaktion mit 2 bis 10 Mol, bevorzugt 2 bis 5 Mol Natriumsulfit in einem Gemisch aus Wasser und einem wasserlöslichen Lösungsmittel, wie 1,4-Dioxan, Methanol oder Ethanol, bevorzugt Wasser/1,4-Dioxan 1:1, abgespalten. Die Reaktionstemperatur beträgt 20 bis 100°C, bevorzugt 80 bis 100°C. Unter diesen Reaktionsbedingungen lassen sich N-Acyldihydroresorufinderivate in hohen Ausbeuten herstellen.

Die Oxidation des Dihydroresorufinteils zu Verbindungen der allgemeinen Formeln Ia und Ib kann mit milden Oxidationsmitteln durchgeführt werden. Bevorzugt wird Kaliumhexacyanoferat-III, welches in 2 bis 6-fachem molarem Überschuß, besonders bevorzugt in 2 bis 4-fachem molarem Überschuß gegenüber dem Leukofarbstoff in einem Gemisch aus Wasser und einem wasserlöslichen Lösungsmittel, wie 1,4-Dioxan, Methanol oder Ethanol, bevorzugt Wasser/Methanol 3:1, verwendet wird. Vorzugsweise erfolgt die Umsetzung in Anwesenheit einer Hilfsbase, wie Natriumhydrogencarbonat oder Natriumcarbonat. Die Reaktionstemperatur beträgt 10 bis 40°C, bevorzugt ist Raumtemperatur.

Besonders vorteilhaft kann die selektive Abspaltung des schützenden N-Acylrestes und die Oxidation des Dihydroresorufinteils in einer Eintopfreaktion erfolgen. Hierzu wird das N-acylierte Dihydroresorufinderivat in Wasser/Methanol 3:1 zunächst mit 2 bis 4 Mol Natriumhydrogencarbonat und einer äquimolaren Menge 1 N Natronlauge und anschließend mit eine 2 bis 4-fachem molarem Überschuß an Kaliumhexacyanoferat-III versetzt. Nach etwa 10 Minuten bis 2 Stunden, bevorzugt 0,5 Stunden bei Raumtemperatur ist die Reaktion abgeschlossen.

In manchen Fällen ist es zweckmäßig, die Verbindungen IIa/IIb nicht direkt mit dem Liganden bzw. dem Ligandenanalogon III, sondern unter Zwischenschalten einer Spacergruppierung $X^3$—M—$X^4$ zu verknüpfen. Als Spacer können alle überlicherweise zu solchen Zwecken eingesetzten Verbindungen mit mindestens zwei reaktiven Gruppen verwendet werden. Besonders bevorzugt sind Diamine oder Aminocarbonsäuren. Die Wahl richtet sich dabei nach der Art der funktionellen Gruppen $X^1$ und $X^2$, die mit dem Spacer verknüpft werden sollen.

Die durch Umsetzung von Resorufinderivaten der allgemeinen Formeln IIa/IIb mit bifunktionellen Verbindungen der allgemeinen Formel IV in einem oder mehreren Schritten, z.B. über Verbindungen der allgemeinen Formel VII resultierenden Verbindungen können durch die allgemeinen Formeln VIIIa und VIIIb

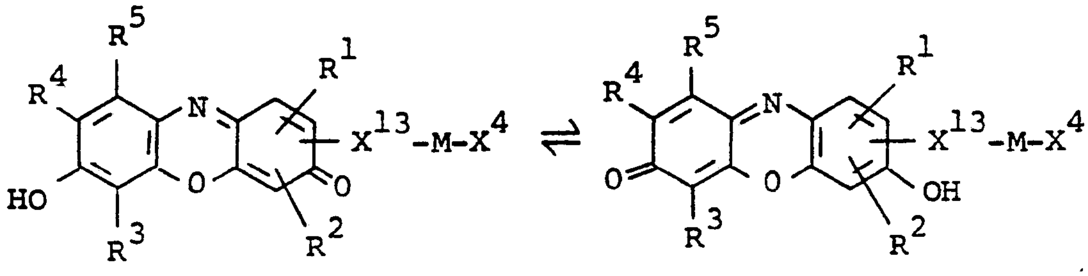

(VIIIa) (VIIIb)

in denen

$R^1$, $R^2$, $R^3$, $R^4$ und $R^5$, die in den allgemeinen Formeln Ia und Ib und

M und $X^4$ die in der allgemeinen Formel IV angegebenen Bedeutungen haben und

$X^{13}$ die dirch Reaktion von $X^1$ und $X^3$ entstehende funktionelle Gruppe vorstellen,

dargestellt werden.

Funktionelle Gruppen $X^{13}$ können alle durch Reaktion der in der Definition von $X^1$ und $X^3$ genannten reaktiven Reste miteinander entstandenen denkbaren Gruppen sein. Bevorzugte Gruppen $X^{13}$ sind Amide, Thioether, Ether, sekundäre oder tertiäre Amine, Harnstoff- oder Thioharnstoffgruppen.

Bedeutet $X^1$ in den allgemeinen Formeln IIa/IIb bzw. VII eine Carbonsäurefunktion bzw. eine hiervon abgeleitete reaktive Gruppe und stellen die reaktiven Gruppen $X^2$ des Liganden bzw. Ligandenanalogons Amino-, Hydroxyl- oder Sulfhydrylgruppen dar, so wird man zweckmäßigerweise einen Spacer $X^3$—M—$X^4$ wählen, wobei $X^3$ eine Aminogruppe und $X^4$ eine Carbonsäurefunktion bzw. ein reaktives Derivat hiervon bedeuten. Das Aminoende kann an die Carbonsäurefunktion der Substanzen IIa/IIb bzw. VII, das Carboxylende an den Liganden oder das Ligandenanalogon $X^2$—A gebunden werden. Stellen jedoch sowohl $X^1$ als auch $X^2$ jeweils eine Carbonsäurefunktion bzw. ein hiervon abgeleitetes aktiviertes Derivat vor, so haben sich Diamine als Spacer bewährt.

Die Umsetzung von Resorufin-Derivaten der allgemeinen Formel II a und II b mit einer bifunktionellen Spacergruppierung IV und dem Liganden bzw. Ligandenanalogon III erfolgt vorteilhaft in zwei Stufen. Zunächst können die Verbindungen IIa/IIb, gegebenenfalls nach vorherigem Überführen in die aktiven Verbindungen VII mit dem Spacer IV verknüpft werden. Die hieraus resultierenden Derivate können dann im zweiten Schritt mit dem Liganden bzw. Ligandenanalogon III umgesetzt werden. Es ist selbstverständlich auch die umgekehrte Vorgehensweise möglich, im ersten Schritt den Spacer IV an den Liganden bzw. an das Ligandenanalogon III zu binden und das erhaltene Produkt im zweiten Schritt mit dem Resorufin-Derivat IIa/IIb bzw. der aktivieren Verbindung VII reagieren zu lassen.

Als Aminocarbonsäuren werden bevorzugt α-Aminocarbonsäuren eingesetzt. Als besonders vorteilhaft haben sich Glycin, Alanin, Sarcosin und Piperidin-4-carbonsäure erwiesen. Die Kupplung von Resorufinderivaten der allgemeinen Formeln IIa/IIb bzw. VII mit Aminocarbonsäuren unter Ausbildung einer Amidbindung erfolgt nach Methoden, die jedem Fachmann geläufig sind. Ganz besonders vorteilhaft ist hierzu der Einsatz von Methyl- oder tert-Butylestern der entsprechenden Aminosäuren. Nach erfolgter Amidbildung werden gegebenenfalls vorher eingeführte Schutzgruppen nach üblichen Methoden selektiv abgespalten. Wird beispielsweise ein aktiviertes Derivat VII mit einer carboxygeschützten Aminocarbonsäure umgesetzt, so ist es erforderlich, die O- und N-Acylgruppen des Dihydroresorufingerüstes selektiv zu hydrolysieren, den Leukofarbstoff wieder zum Resorufinsystem zu oxidieren und abschließend die Carboxyschutzgruppe der begundenen Aminocarbonsäure unter üblichen Bedingungen, bevorzugt mit Trifluoressigsäure, abzuspalten. Die freie Carboxylgruppe kann dann zur Konjugation eines Liganden oder Ligandenanalogons III in entsprechender Weise, wie für Resorufinderivate der allgemeinen Formeln IIa und IIb beschrieben, aktiviert werden. Auch die Durchführung der Konjugation selbst erfolgt analog, wie für die direkte Konjugation von Liganden oder Ligandenanaloga an Resorufinderivate der allgemeinen Formeln IIa und IIb ausgeführt.

Im Falle carboxylgruppenhaltiger Liganden oder Ligandenanaloga ist es zweckmäßig, Resorufinderivate der allgemeinen Formeln IIa/IIb bzw. VII in aminogruppenhaltige Verbindungen überzuführen, die sich dann mit den carboxylgruppenhaltigen Liganden bzw. Ligandenanaloga unter Amidbindungsbildung umsetzen lassen. Hierzu hat sich als besonders einfach und vorteilhaft die Umsetzung von Verbindungen IIa/IIb bzw. VII mit Diaminen (Formel IV mit $X^3$ und $X^4$ Aminogruppe) erwiesen. Bevorzugte Diamine in diesem Sinne sind z.B. Piperazin, 1,2-Diaminoethan oder 1,3-Diaminopropan.

Die Überführung von Resorufinderivaten der allgemeinen Formeln IIa/IIb bzw. VII mit Diaminen in Derivate mit freier Aminogruppe erfolgt nach Methoden, die jedem Fachmann geläufig sind. Um besonders hohe Ausbeuten an monosubstituierten Diaminen zu erzielen, werden bevorzugt solche Diamine zur Reaktion gebracht, die nur eine reaktive Aminogruppe besitzen und deren zweite funktionelle Gruppe durch eine Schutzgruppe blockiert ist. Grundsätzlich sind alle üblichen Aminoschutzgruppen verwendbar, die ohne Beeinträchtigung der Amidbindungen wieder abgespalten werden können. Als besonders vorteilhaft hat sich die Verwendung der t-Butoxycarbonyl- bzw. der Benzoyl-oxy-carbonyl-Schutzgruppe erwiesen.

Nach der Reaktion monogeschützter Diamine mit Resorufinderivaten der allgemeinen Formeln IIa/IIb bzw. VII werden gegebenenfalls eingeführte Schutzgruppen wieder abgespalten und gegebenenfalls als Zwischenstufe auftretender Leukofarbstoff zum Resorufinsystem oxidiert. Die Abspaltung der Aminoschutzgruppe des gebundenen Diamins erfolgt hierbei unter üblichen Bedingungen, bevorzugt mit Trifluoressigsäure.

Die so erhaltenen aminogruppenhaltigen Resorufinderivate können in üblicher Weise mit Liganden oder Ligandenanaloga III, die als Reaktive Gruppen $X^2$ Carboxylgruppen besitzen, konjugiert werden. Vorteilhafterweise sollen diese Carboxylgruppen aktiviert sein. Dies kann in der oben beschriebenen Weise erfolgen. Bevorzugt ist der Einsatz von Liganden oder Ligandenanaloga der allgemeinen Formel III, in der $X^2$ aktivierte Estergruppen bedeuten. Als besonders vorteilhaft haben sich vor allem N-Hydroxysuccinimidester erwiesen. Die Durchführung der Konjugation selbst erfolgt grundsätzlich analog der oben für die direkte Konjugation von Resorufinderivaten mit Liganden oder Ligandenanaloga beschriebenen Vorgehensweise unter Berücksichtigung der vertauschten Rollen der funktionellen Gruppen.

Vorzugsweise erfolgt die weitere Umsetzung der Produkte, die nach der Verknüpfung von Resorufinderivaten IIa/IIb bzw. VII mit einem Zwischenglied $X^3$—M—$X^4$ der Formel IV erhalten werden, mit niedermolekularen Liganden $X^2$—A, z.B. Haptenen, in einem Gemisch aus Wasser oder Puffer und einem wasserlöslichen Lösungsmittel, wie z.B. 1,4-Dioxan, Methanol oder Ethanol. Bevorzugt wird die Mischung 0,1 M Kaliumphosphatpuffer, pH 8,5/1,4-Dioxan im Verhältnis 1:1. Es ist möglich, den Reaktionsverlauf dünnschichtchromatographisch zu verfolgen. Die Reaktionsdauer kann zwischen 1 bis 24 Stunden betragen, meist ist die Reaktion bereits nach 1 bis 18 Stunden vollständig abgelaufen.

Für die Umsetzung von Resorufinderivaten IIa/IIb bzw. VII oder VIIIa/VIIIb, mit hochmolekularen Liganden der allgemeinen Formel III erweisen sich N-Hydroxy-succinimidester als besonders vorteilhaft. So reichen z.B. bei der Kupplung an Kaninchen-IgG bereits 6 Mol Resorufinderivat pro Mol IgG, um einen Markierungsgrad von 3 Molekülen Resorufin pro Mol IgG zu erreichen. Die bisher hierzu üblichen Fluoreszenzfarbstoffe, die eine maximale Absorptionswellenlänge $\lambda_{max}$ >470 nm aufweisen, enthalten als reaktive Gruppe einen Isothiocyanat- oder Sulfonsäurechlorid-Rest, z.B. Fluoresceinisothiocyanat oder Texas Rot. In diesen Fällen muß zur Kupplung an Kaninchen IgG ein wesentlich höherer Farbstoffüberschuß eingesetzt werden, um den gleichen Markierungsgrad zu erreichen.

Die Kunjugation hochmolekularer Verbindungen, der allgemeinen Formel III wie z.B. von Proteinen, erfolgt bevorzugt in Puffer, besonders vorteilhaft in 0,1 M Kaliumphosphatpuffer, pH 8,0 bis 9,0, wenn die reaktive Gruppe $X^1$ oder $X^2$ ein N-Hydroxysuccinimidester ist. Die Reaktionstemperatur kann jeweils 10 bis 35°C betragen. Bevorzugt wird die Reaktion bei Raumtemperatur durchgeführt.

Aufgrund ihrer besonders günstigen spektralen Eigenschaften ist ein weiterer Gegenstand der Erfindung die Verwendung der erfindungsgemäßen Verbindungen der allgemeinen Formeln Ia und Ib in analytischen Verfahren, bei denen eine Fluoreszenseigenschaft der Verbindungen der allgemeinen Formel Ia/Ib bzw. verfahrensbedingt entstandener Umwandlungsprodukte gemessen wird.

In heterogenen Immunoassays ist eine Trennung von an Antikörper gebundenen Liganden und freien Liganden durch Fällung mit geeigneten Substanzen oder durch Verwendung von an feste Träger gebundenen Antikörpern erforderlich, bevor entweder die Konzentration freier oder gebundender Liganden bestimmt wird. Im homogenen Immunoassays erfolgt die Untersuchung der Antikörper-Liganden-Komplexbildung in der Probe ohne eine solche Abtrennung. Zu den homogenen Immunoassay-Methoden sind beispielsweise Fluoreszenz-Quenching-, Fluoreszenz-Enhancement- under Fluoreszenzpolarisations-Methoden zu zählen, bei denen fluoreszierende Substanzen als Markierungsmittel verwendet werden. Besonders die letztgenannte Methode litt bisher unter den in der Einleitung bereits genannten Nachteilen der verwendeten gebräuchlichen Fluoreszenzmarker. Da die erfindungsgemäßen Verbindungen der allgemeinen Formeln Ia oder Ib Absorptions- und Emissionsmaxima besitzen, die weitgehend außerhalb des in Körperflüssigkeiten durch seine Eigenfluoreszenz störenden biologischen Materials liegen, sind die besonders zur Verwendung in Fluoreszenzpolarisations-Immunoassays (FPIA) geeignet. Ein weiterer Vorteil der erfindungsgemäßen Verbindungen liegt darin, daß sie bei geeigneter Substitution einen besonders hohen Stokes-Shift bis zu etwa 70 nm aufweisen.

FPIA-Verfahren basieren grundsätzlich auf dem Prinzip üblicher Fluoreszenz-Immunoassays.

Werden geeignete fluoreszenzmarkierte Liganden mit linear polarisiertem Licht zur Fluoreszenz angeregt, so kann aufgrund der geringen zeitlichen Verzögerung zwischen Anregung und Emission, das Molekül, bevor es Strahlung emittiert, rotieren. Damit wird die Ebene des linear polarisierten Lichts ebenfalls um einem bestimmten Winkel gedreht. Eine Vielzahl von Molekülen kann innerhalb dieses kurzen Zeitraumes durch Rotationsdiffusion zu einer gewissen Depolarisation der Fluoreszenzemission führen. Für die Polarisation der emittierten Fluoreszenz gilt, daß sie um so größer ist, je größer das Molekül, und infolgedessen je langsamer die Rotation ist. Diesen Zusammenhang kann man für die Messung der Bindung von Liganden an Antikörper nutzen, denn freie, markierte Liganden besitzen ein kleineres Molekülvolumen als Komplexe aus an Antikörper gebundenen, markierten Liganden. Die Polarisation ist umgekehrt proportional zu der in der Probe befindlichen zu bestimmenden Ligandenkonzentration.

Die Konzentration des markierten Liganden oder Ligandenanalogons und des für solche immunologischen Verfahren erforderlichen Antikörpers richten sich nach der verwendeten Meßapparatur sowie nach den jeweiligen charakteristischen Eigenschaften des verwendeten markierten Liganden oder Ligandenanalogons und des Antikörpers selbst Grundsätzlich hängen diese Konzentrationen natürlich auch von der Konzentration des zu bestimmenden Liganden ab und müssen deshalb empirisch festgelegt werden. Diese Festlegung kann von jedem Fachmann durch einfache Optimierung getroffen werden.

Die Ligandenkonzentration die bestimmt werden soll, schwankt im allgemeinen von etwa $10^{-2}$ bis etwa $10^{-13}$ molar. Vorteilhaft ist es, für die Messung eine Ligandenkonzentration in der zu bestimmenden Probe von etwa $10^{-3}$ bis etwa $10^{-12}$ molar, besonders vorteilhaft von etwa $10^{-4}$ bis etwa $10^{-10}$ molar einzustellen. Höhere Ligandenkonzentrationen können nach Verdünnung der ursprünglichen Probe gemessen werden.

Die Messung erfolgt bei bestimmten pH-Werten, welche von etwa 3 bis 12 reichen können. Gewöhnlich liegen sie im Bereich von etwa 5 bis etwa 10, vorzugsweise im pH-Bereich von etwa 6 bis etwa 9. Zur Erzielung und Aufrechterhaltung des pH-Wertes während der Messung können unterschiedliche Puffer verwendet werden, wie z.B. Borat-, Phosphat-, Carbonat- oder Tris-Puffer. Welcher Puffer verwendet wird, ist für die vorliegende Erfindung nicht entscheidend. Die Auswahl richtet sich in erster Linie nach dem eingesetzten Antikörper, nach dem Liganden, der bestimmt werden soll, sowie dem eingesetzten Fluoreszenzmarker.

Vorzugsweise wird die FPIA-Methode bei konstanter Temperatur durchgeführt. Normalerweise kann eine Temperatur im Bereich von etwa 0 bis 50°C, vorzugsweise im Bereich von etwa 10 bis etwa 40°C gewählt werden.

Der genaue Zusammenhang zwischen Polarisation und Konzentration eines zu bestimmenden Liganden oder Ligandenanalogans ist jeweils aus Eichkurven ablesbar. Diese werden erhalten, indem die Polarisiationswerte von Lösungen unterschiedlicher aber bekannter Konzentrationen entsprechender Substanzen gemessen werden. Unbekannte Ligandenkonzentrationen einer zu untersuchenden Probe können dann bei Kenntnis der Polarisation aus solchen Eichkurven ermittelt werden.

Ein breites Anwendungsgebiet der erfindungsgemäßen Verbindungen der allgemeinen Formeln Ia und Ib ist ihrer allgemeinen Verwendbarkeit als Fluoreszenzmarker zu sehen. So können z.B. in der Immunofluoreszenz-Mikroskopie als Antigene Proteine oder ganze Zellen durch fluoreszenzmarkierte Antikörper sichtbar gemacht werden. In entsprechender Weise kann auch die Vertailung eines Haptens oder Antigens in einer Zelle direkt beobachtet werden, wenn die betreffende Verbindung fluoreszenzmarkiert in die Zelle eingebracht und z.B. unter dem Mikroskop verfolgt wird. Hierbei ändern sich, im Gegensatz zu den vorstehend beschriebenen homogenen Fluoreszenzimmunoassays die Fluoreszenzeigenschaften der Resorufinderivate nicht.

Bekannte Farbstoffe, welche bisher als Fluoreszenzmarker verwendet wurden, sind beispielsweise Fluoresceine, wie Fluoresceinisothiocyanat oder Rhodaminfarbstoffe, wie Texas Rot. Wie bereits zuvor ausgeführt, haben Fluoresceine jedoch den Nachteil, daß sie bei relativ niedrigen Wellenlängen fluoreszieren. Außerdem ist die Ausbeute der Kupplungsreaktion an den jeweiligen Träger erfahrungsgemäß meist gering und zusätzlich ist die Farbstabilität der Kupplungsprodukte schlecht. Gleiches gilt auch für Rhodaminfarbstoffe.

Gerade in Bezug auf diese Punkte zeigen die erfindungsgemäßen Verbindungen der allgemeinen Formeln Ia und Ib deutliche Vorteile gegenüber den im Stand der Technik bekannten Fluoreszenzmarkern. Sie fluoreszieren langwellig bei guter Farbstabilität und sie sind in guter Ausbeute aus Verbindungen der allgemeinen Formeln IIa oder IIb und entsprechenden Kupplungspartnern herstellbar.

Die erfindungsgemäßen Verbindingen der allgemeinen Formel IIa oder IIb lassen sich natürlich auch zur Markierung von Substanzen in anderen als den genannten fluoreszenzimmunologischen Verfahren verwenden. So ist auch die Markierung eines Partners eines anderen komplexbildenden Systems mit diesen reaktiven Resorufinverbindungen möglich. Unter komplexbildenden Systemen können hierbei alle diejenigen Kombinationen von Substanzen verstanden werden, die aufgrund spezifischer Wechselwirkungskräfte in der Lage sind, Komplexe zu bilden. Bekannte Kombinationen sind z.B. Hormon/spez. Rezeptor, Biotin/Avidin, Kohlenhydratderivat/Lectin. Beispielsweise können mit Biotin markierte Proteine mittels eines Kupplungsproduktes aus Avidin und einer erfindungsgemäßen reaktiven Verbindung der allgemeinen Formel IIa oder IIb bestimmt werden. Eine weitere vorteilhafte Anwendbarkeit erfindungsgemäßer Verbindungen der allgemeinen Formel Ia und Ib ist bei Bestimmungen eines Partners des Systems Lectin/Kohlenhydratderivat gegeben.

Zur Sortierung von Zellen im "Fluorescent Activated Cell Sorter" finden fluoreszenzmarkierte Latexpartikel Anwendung. Solche Teilchen können ebenfalls sehr gut, z.B. durch Reaktion hydroxyl-, sulfhydryl-, amino- oder auch carboxyl- oder sulfonsäuregruppenhaltiger Latexpatikel mit reaktiven Resorufinverbindungen der allgemeinen Formeln IIa oder IIb fluoreszenzmarkiert werden.

Verbindungen der allgemeinen Formeln Ia und Ib lassen sich ebenfalls vorteilhaft zur Bestimmung von Enzymen einsetzen. Hierzu kann ein Resorufinderivat der allgemeinen Formel IIa oder IIb an ein Substrat gebunden werden, welches durch das zu bestimmende Enzym spaltbar ist. Auch diese Kupplungsprodukt ist eine erfindungsgemäße Verbindung der allgemeinen Formel Ia und Ib. Nach Umsetzung des resorufinmarkierten Substrats mit dem Enzym und Trennung von Spaltprodukten und unumgesetztem Substrat kann die Aktivität des Enzyms bestimmt werden. Beispielsweise kann ein reaktives Resorufinderivat der allgemeinen Formel IIa oder IIb an ein Glycopeptid gebunden und das hierdurch erhaltene Kupplungsprodukt als Substrat zum Nachweis von Endoglucosidase-Aktivität verwendet werden. Zur Bestimmung von Endoglucosidasen ist die Markierung mit Dansylverbindungen bekannt (Iwase et al., Anal. Biochem. *113,* 93—95 [1981]). Resorufinderivate zeichen sich im Vergleich mit solchen Verbindungen besonders durch ihre höhere Empfindlichkeit aus.

Die folgenden Beispiele dienen zur Verdeutlichung der Erfindung. Sie sollen die grundsätzliche Möglichkeit der Markierung nieder- oder hochmolekularer Verbindungen und deren Verwendung zeigen.

### Beispiel 1

### Resorufin-4-carbonsäure-[3-(1-diphenylhydantoinyl)ethyl-carbonyl]-piperazid

(a) Resorufin-4-carbonsäure

16 g Nitrosoresorcin, 15,5 g 2,6-Dihydroxybenzoesäure und 8,6 g Braunstein werden in 200 ml Methanol suspendiert und auf 0°C gekühlt. Dazu tropft man 10,6 ml konz. Schwefelsäure und rührt dann noch 2 Stunden bei Raumtemperatur. Die ausgefallene rote Resazurin-4-carbonsäure wird abfiltriert, mit Methanol gewaschen und getrocknet.

Das Resazurinderivat wird in 200 ml Wasser und 50 ml 25 %igen Ammoniak aufgenommen und filtriert. Zu dem blauen Filtrat gibt man unter Eiskühlung portionsweise 50 g Zinkstaub und läßt auf Raumtemperatur erwärmen. Der Reduktionsverlauf läßt sich leicht dünnschichtchromatographisch verfolgen (Fließmittel: Methanol/Essigester 1:1; Kieselgel DC-Platten). Die Reaktionslösung wird filtriert, danach säuert man das Filtrat mit Eisessig und wenig konz. Salzsäure an. Die ausgefallene Resorufin-4-carbonsäure wird abfiltriert und über Phosphorpentoxid im Vakuum getrocknet.

Ausbeute: 16,33 g.

Rf (Kieselgel, Fließmittel: n-Butanol/Eisessig/Wasser 4:1:1) = 0.4.

b) N,O,O-Triacetyldihydroresorufin-4-carbonsäure

12,9 g Resorufin-4-carbonsäure werden in 30 ml Eisessig und 30 ml Acetanhydrid aufgenommen, mit 27,6 g Zinn-II-chlorid versetzt und 1 Stunde bei 80°C gerührt. Man gibt auf 600 ml Eiswasser, rührt 1 Stunde und filtriert den Niederschlag ab. Nach Trocknen wird der Feststoff in 500 ml Aceton aufgenommen. Man saugt ab, dampft das Filtrat ein und erhält nach Trocknen 11,3 g Produkt.

$^1$H-NMR([$D_6$]-DMSO: δ=2.24, 2.26 und 2.29 (je s, 9H); 6.94 (dd, J=8.5 und 2.2Hz, 1H); 6.98 (d, J=2.2Hz, 1H); 7.04 (d, J=9Hz, 1H); 7.61 (d, J=8,5Hz, 1H); 7.67 ppm (d, J=9Hz, 1H).

Rf (Kieselgel, Fließmittel: Chloroform/Methanol/Eisessig 9:1:0,1): 0.46.

c) N,O,O-Triacetyldihydroresorufin-4-carbonsäurechlorid

38,5 g des Beispiel 1b) beschriebenen Triacetats werden mit 54 ml Oxalylchlorid verstzt und auf 0°C gekült. Dazu gibt man einem Tropfen Dimethylformamid und läßt auf Raumtemperatur erwärmen. Das Edukt löst sich dabei unter Gasentwicklung. Man dampft im Vakuum zur Trockne ein, nimmt je 3 mal mit 200 ml trockenem Methylenchlorid auf und dampft wiederum zur Trockne ein.

Ausbeute: 41 g.

d) N-BOC-Piperazin

12,61 g N-Benzhydrylpiperazin (EMKA-Chemie) werden in 100 ml 1,4-Dioxan/Wasser 3:1 aufgenommen. Dazu tropft man 12,0 g Di-tert-butyldicarbonat, gelöst in 50 ml 1,4-Dioxan. Nach 1/2 Stunde Rühren tropft man 50 ml Wasser zu, filtriert und trocknet den Niederschlag.

Ausbeute: 16,2 g N-BOC-N'-Benzhydrylpiperzin.

RF (Kieselgel, Laufmittel: Chloroform/Methanol/Eisessig 9:1:0,1): 0,92.

7 g N-BOC-N'-Benzhydrylpiperazin werden in 100 ml Essigester und 5 ml Eisessig aufgenommen. Mit 0,3 g Palladium auf Aktivkohle wird hydriert, danach vom Katalysator filtriert und eingedampft. Der Rückstand wird mit 100 ml Wasser und 20 ml 1 N HCl versetzt und filtriert. Man extrahiert das Filtrat zweimal mit Essigester und macht dann die wässrige Phase mit Natronlauge basisch. Das ölig ausgefallene Produkt wird mit Dichlormethan extrahiert. Nach Trocknen mit Natriumsulfat und Eindampfen erhält man 3,2 g N-BOC-Piperazin als Öl, das nach einigen Tagen vollständig kristallisiert.

Rf (Kieselgel, Laufmittel: Chloroform/Methanol/Eisessig 9:1:0,1): 0,05, wird mit Ninhydrin blau.

e) N,O,O-Triacetyldihydroresorufin-4-carbonsäure-(N'-BOC-piperazid)

Zu 25 g des Beispiel 1c) beschriebenen Säurechlorids und 17,3 ml Triethylamin in 450 ml Dichlormethan tropft man bei 0°C eine Lösung von 13,8 g N-BOC-Piperazin in 50 ml Dichlormethan. Man rührt noch 1 Stunde ohne Kühlung, schüttelt dreimal mit Wasser aus und dampft die organische Phase ein.

Ausbeute: 36,0 g.

Rf (Kieselgel, Laufmittel: Chloroform/Methanol/Eisessig 9:1:0,1) = 0,64.

f) N-Acetyldihydroresorufin-4-carbonsäure-(N'-BOC-piperazid)

34,3 g des in Beispiel 1e) beschriebenen Triacetats und 17,1 g Natriumsulfit werden 1 Stunde bei 60°C in 500 ml 1,4-Dioxan/Wasser 1:1 gerührt. Man dampft anschließend ein, nimmt in Esigester auf, filtriert von den unlöslichen Salzen und chromatographiert an 2 l Kieselgel (Eluens: Essigester/Dichloromethan 4:1, sobald das Produkt eluiert wird, wird auf reinen Essigester umgestellt).

Ausbeute: 14 g.

Rf (Kieselgel, Laufmittel: Essigester/Dichlormethan 4:1): 0,28.

g) Resorufin-4-carbonsäure-(N'-BOC-piperazid)

5 g der in Beispiel 1f) beschriebenen N-Acetylverbindung werden in 200 ml Methanol und 600 ml Wasser gelöst. Man gibt 1,8 g Natriumhydrogencarbonat und 10,7 ml 1 N NaOH zu, danach 14 g Kalumhexacyanoferrat-III. Nach 1/2 Stunde Rühren bei Raumtemperatur stellt man den pH auf 5. Das Produkt fällt aus und wird abgesaugt.

Ausbeute: 2,72 g.

Rf (Kieselgel, Laufmittel: Chloroform/Methanol/Eisessig 9:1:0,1): 0,28.

h) Resorufin-4-carbonsäurepiperazid-trifluoracetat

1 g des in Beispiel 1g) beschriebenen BOC-Derivats werden 15 min in 20 ml Trifluoressigsäure stehengelassen. Man dampft ein digeriert mit Ether und filtriert das Produkt ab.

Ausbeute: 0,96 g.

Rf (Kieselgel, Laufmittel: Chloroform/Methanol/Eisessig 9:1:0,1): 0,02.

i) Kopplung von Resofurin-4-carbonsäurepiperazid mit 3-(1-Diphenylhydantoinyl)-propionsäure-N-hydroxysuccinimid-ester

191 mg des in Beispiel 1h) beschriebenen Piperazid-trifluoracetats und 210 mg 3-(1-Diphenylhydantoinyl)propionsäure-N-hydroxysuccinimidester (hergestellt aus Diphenylhydantoin-Natrium-Salz und 3-Brompropionsäureethylester analog Cook et al., Res. Communications in Chemical Pathology and Pharmacology *5* (1973), S. 767) werden 15 Stunden in 20 ml Dioxan und 20 ml 0,1 M Kaliumphosphatpuffer pH 8,5 gerührt. Man filtriert vom ausgefallenen Produkt, dampft das Filtrat ein und chromatographiert an Kieselgel RP18 (Eluens: Isopropanol), wobei zusätzlich Produkt gewonnen wird. Man kristallisiert aus Essigester/Methanol und erhält insgesamt 250 mg Kopplungsprodukt.

Rf (Kieselgel, Laufmittel: Chloroform/Methanol/Eisessig 9:1:0,1): 0,61.

$^1$H-NMR([$D_6$]-DMSO): δ=2.6—2.8 (m, 2H); 3.0—3.8 (m, 10H); 6,74 (d, J=2.2Hz, 1H); 6.82 (d, J=9.5Hz, 1H); 6,91 (dd, J=9.5 und 2.2Hz); 7.25—7.33 (m, 10H); 7,55 und 7,66 ppm (je, d, J=9.5Hz, 2H).

UV/VIS(0,1 M Kaliumphosphatpuffer pH 7,5): $\lambda_{max}$ = 576,8 nm.

Fluoreszenzemission : $\lambda_{max}$ = 592 nm.

Beispiel 2

Kopplung von Resorufin-4-carbonsäurepiperazid mit 3-(1-Diphenylhydantoinyl)essigsäure-N-hydroxysuccinimidester

Analog Beispiel Ii) erhält man aus 365 mg Resorufin-4-carbonsäurepiperazid-trifluoracetat und 339 mg 3-(1-Diphenylhydantoinyl)essigsäure-N-hydroxysuccinidester 210 mg gewünschtes Produkt.

Rf (Kieselgel, Fließmittel: n-Butanol/Eisessig/Wasser 4:1:1): 0,82.

$^1$H-NMR([$D_6$]-DMSO): δ = 3.2—4.5 (m, 10H); 6.60 (d, J=2.4Hz, 1H); 6.71 (d, J=9.5Hz, 1H); 6.80 (dd, J=9.05 und 2.4Hz, 1H); 7.39 ("s", 10H); 7.52 (d, J=9.5Hz, 1H); 7.61'(d, J=9.0Hz, 1H); 9.65 ppm (s, 1H).

UV/VIS (0.1 M Kaliumphosphatpuffer, pH 8,0): $\lambda_{max}$ = 575,4 nm.

Fluoreszenzemission: $\lambda_{max}$ = 592 nm.

Beispiel 3

Kopplung von Resorufin-4-carbonsäurepiperazid mit N-BOC-L-Thyroxin-N-hydroxysuccinimidester

Analog Beispiel Ii) erhält man aus 212 mg Resorufin-4-carbonsäurepiperazid-trifluoracetat und 419 mg N-BOC-L-Thyroxin-N-hydroxysuccinimidester 320 Produkt.

Rf (Kieselgel, Fließmittel: Chloroform/Methanol/Eisessig 9:1:0,1): 0,58 Den N-BOC-L-Thyroxin-N-hydroxysuccinimidester erhält man auf folgende Weise:

a) N-BOC-Thyroxin

Die Lösung von 10 g (12,5 mmol) L-Thyroxin-Na-Salz $H_2O$ in einem Gemisch aus 300 ml Dioxan/Wasser = 2/1 und 15 ml 1 N Natronlauge wird mit 3 g (13,75 mmol) Di-tert.-butyl-dicarbonat $(BOC)_2O$ versetzt und 2 Std. unter Lichtausschluß bei Raumtemperatur gerührt.

Mit 2 M $KHSO_4$-Lsg. wird auf pH 2 gestellt, mit Essigester extrahiert, der Essigester-Extrakt mit Wasser gewaschen, über Natriumsulfat getrocknet und eingedampft. Der feste Rückstand wird mit Petrolether angerieben, abgesaugt und im Exsikkator getrocknet.

Ausbeute: 9,45 g = 86% d. Th.

Rf (Kieselgel, Laufmittel: $CHCL_3$/Ligroin/Essigsäure = 6:3:1): = 0,6.

b) N-BOC-Thyroxin-N-hydroxysuccinimidester

Zu einer Lösung 8,8 g L-BOC-Thyroxin in 200 ml Ethylenglykoldimethylether werden 1,2 g (9,5 mmol) N-Hydroxysuccinimid gegeben. Die Lösung wird auf 10°C abgekühlt und tropfenweise mit einer Lösung von 2,3 g (9,9 mmol) Dicyclohexylcarbodiimid in 40 ml Ethylenglykoldimethylether versetzt. Nach 2 Stunden Rühren bei Raumtemperatur wird der ausgefallene Dicyclohexylharnstoff abgesaugt und das Filtrat im Vakuum bei 40°C eingedampft. Der Rückstand wird mit Isopropanol angerieben und abgesaugt. Im Exsikkator wird bei Raumtemperatur getrocknet.

Ausbeute: 9,19 g = 94% d. Th. (Gesamtausbeute bez. auf Thyroxin = 81%).

Rf (HPTCL-RP 18; Laufmittel: Nitromethan/Ethanol = 9:1): 0,8; oder (HPTCL-RP 18; Laufmittel: Acetonitril/$H_2O$ = 8:2): 0,6.

$^1$H-NMR([$D_6$]-DMSO): δ = 1,36 (s, 9H); 2,81 (s, 4H); 2,9—3,2 (m, 2H); 4,5—4,9 (m, 1H); 7,03 (s, 2H); 7,63 (d, j = 9Hz, 1H); 7,90 (s, 2H); = 9,2 (s, 1H).

Beispiel 4

Kopplung von Resorufin-4-carbonsäurepiperazid mit 3-O-[3-(N-Succinimidoxycarbonyl)propyl]östradiol

Analog Beispiel Ii) erhält man aus 212 mg Resorufin-4-carbonsäurepiperazid-trifluoracetat und 220 mg 3-O-[3-(N-Succinimidyloxycarbonyl)propyl]östradiol 295 mg Produkt.

Rf (Kieselgel, Fließmittel: Chloroform/Methanol/Eisessig 9:1:0,1): 0,58.

3-O-[3-(N-Succinimidoxycarbonyl)propyl]östradiol erhält man in üblicher Weise aus 3-O-Carboxypropyl-östradiol (aus Östradiol und Brombuttersäure analog Lübke et al. in Immunologische Teste für niedermolekulare Wirkstoffe, G. Thieme Verlag, Stuttgart, S. 94) und N-Hydroxysuccinimid in Gegenwart von Dicyclohexylcarbodiimid.

Beispiel 5

Kopplung von Resorufin-4-carbonsäurepiperazid mit N-[3-(N-Succinimidoxycarbonyl)propyl]phenobarbital

Analog Beispiel Ii) erhält man aus 212 mg Resorufin-4-carbonsäurepiperazid-trifluoracetat und 205 mg N-[3-(N-Succinimidoxycarbonyl)propyl]phenobarbital 220 mg Produkt.

Rf (Kieselgel, Fließmittel: Chloroform/Methanol/Eisessig 9:1:0,1): 0,45.

N-[3-(N-Succinimidoxycarbonyl)propyl]phenobarbital erhält man in üblicher Weise aus Phenobarbital-1-buttersäure (T. Nistikawa et alk. Clin. Chem. Acta *91* (1979) S. 59) und N-Hydroxysuccinimid in Gegenwart von Dicyclohexylcarbodiimid.

Beispiel 6

Kopplung von Resorufin-4-carbonsäurepiperazid mit Theophyllin-7-propionsäure-N-hydroxysuccinimidester

Aus 212 mg Resorufin-4-carbonsäurepiperazid-trifluoracetat und 175 mg Theophyllin-7-propionsäure-N-hydroxysuccinimidester erhält man analog Beispiel Ii) 200 mg Produkt.

Rf (Kieselgel, Fließmittel: Chloroform/Methanol/Eisessig 9:1:0,1): 0,44.

Theophyllin-7-propionsäure-N-hydroxysuccinimidester erhält man in üblicher Weise aus Theophyllin-7-propionsäure (T. Nistikawa et al., Chem. Pharm. Bull. *27* (1979) S. 893) und N-Hydroxysuccinimid in Gegenwart von Dicyclohexylcarbodiimid.

Beispiel 7

Kopplung von N-(4-Resorufinylcarbonyl)sarcosin-N'-hydroxysuccinimidester mit 1-(2-Aminoethyl)diphenyl-hydantoin

a) N,O,O-Triacetyldihydroresorufin-4-carbonsäure(tert-butoxycarbonylmethyl)methylamid

10 g des in Beispiel 1c) beschriebenen Säurechlorids werden analog Beispiel 1e) mit Sarcosin-tert.-butylester umgesetzt.

Ausbeute: 7,5 g.

Rf (Kieselgel, Fließmittel: Chloroform/Methanol/Eisessig 9:1:0,1): 0,77.

b) N-Acetyldihydroresorufin-4-carbonsäure(tert-butoxycarbonylmethyl)methylamid

7,5 g des Beispiel 7a) beschriebenen Produkts werden analog Beispiel 1f) deacetyliert.

Ausbeute: 5,2 g.

Rf (Kieselgel, Fließmittel: Chloroform/Methanol/Eisessig 9:1:0,1): 0,56.

c) Resorufin-4-carbonsäure(tert-butoxycarbonylmethyl)methylamid

4,5 g des in Beispiel 7b) beschriebenen Produkts werden analog Beispiel 1g) umgesetzt.

Ausbeute: 2,6 g.

Rf (Kieselgel, Fließmittel: Chloroform/Methanol/Eisessig 9:1:0,1): 0,64.

d) Resorufin-4-carbonsäure(carboxymethyl)methylamid

0,55 g des in Beispiel 7c) beschriebenen Produkts werden 1 Stunde bei Raumtemperatur in 6 ml Trifluoressigsäure stehengelassen. Man dampft zur Trockne ein, reibt mit Ether an und filtriert.

Ausbeute: 0,45 g.

Rf (Kieselgel, Fließmittel: Chloroform/Methanol/Eisessig 9:1:0,1): 0,11.

e) N-(4-Resorufinylcarbonyl)sarcosin-N'-hydroxysuccinimidester

200 mg des Produkts aus Beispiel 7d) werden mit 72 mg N-Hydroxysuccinimid und 138 mg Dicyclohexylcarbodiimid 14 Stunden in 40 ml Tetrahydrofuran gerührt. Man filtriert vom ausgefallenen Harnstoff, dampft ein und chromatographiert an Kieselgel RP 18 (Eluens: Nitromethan/Ethanol 4:1).

Ausbeute: 150 mg.

Rf (Kieselgel RP-18, Fließmittel: Nitromethan/Ethanol 4:1): 0,79.

f) Kopplung von N-(4-Resorufinylcarbonyl)-sarcosin-N'-hydroxysuccinimidester mit 1-(2-Aminoethyl)diphenylhydantoin

125 g des N-Hydroxysuccinimidester aus Beispiel 7e) werden mit 90 mg 1-(2-Aminoethyl-diphenylhydantoin in 40 ml Dioxan/Kaliumphosphatpuffer, pH 8,5, 1:1 1 Stunde gerührt. Man dampft das Dioxan ab, gibt bis zum vollständigen Farbumschlag Ammoniak zu, filtriert und fällt das Produkt mit HCl aus dem Filtrat.

Ausbeute: 110 mg.

Rf (Kieselgel, Fließmittel: n-Butanol/Eisessig/Wasser 4:1:1): 0,78.

UV/VIS (0,1 M Kaliumphosphatpuffer, pH 8,0): $\lambda_{max}$ = 575 nm.

Fluoreszenzemission: $\lambda_{max}$ = 592 nm.

Beispiel 8

Resorufin-4-carbonsäure-2(1-diphenylhydantoinyl)ethylamid

a) N,O,O-Triacetyldihydroresorufin-4-carbonsäure-2(1-diphenylhydantoinyl)ethylamid

Analog Beispiel 1e) werden 1,37 g 1-(2-Aminoethyl)diphenylhydantoin mit 1,2 g N,O,O-Triacetyldihydroresorufincarbonsäurechlorid umgesetzt. Man erhält 1,9 g Produkt als leicht gefärbten Schaum.

b) Resorufin-4-carbonsäure-2(1-diphenylhydantoinyl)ethylamid

Das in Beispiel 8a) erhaltene Produkt wird analog den Beispielen 1f) und 1g) oxidativ deacetyliert. Aus 1,9 g Edukt erhält man 600 mg Produkt.

Rf (Kieselgel, Fließmittel: Chloroform/Methanol/Eisessig 9:1:0,1): 0,68.

$^1$H-NMR ([$D_6$]-DMSO): δ = 3/2—3.6 (m, 4H); 6.73 (d, J=2.2Hz, 1H); 6.84 (d, J=9.5Hz, 1H); 6.86 (dd, J=9.5 und 2.2Hz, 1H); 7.2—7.4 (m, 10H); 7,62 und 7,66 (je, d, J=9.5Hz, 2H); 8.66 (t, breit, J=5Hz, 1H); 9.58 ppm (s, 1H).

UV/VIS (0,1 M Kaliumphosphatpuffer, pH 8.0) $\lambda_{max}$ = 575 nm.

Fluoreszenzemission: $\lambda_{max}$ = 591 nm.

Beispiel 9

Kupplung von 6-Methylresorufin-4-carbonsäure-piperazid mit [2-(1-Diphenylhydantoinyl)essigsäure-N-hydroxysuccinimidester

a) 2-Methyl-4-nitrosoresorcin

19,8 g 2-Methylresorcin und 13,4 g Kaliumhydroxid werden in 120 ml Ethanol gelöst und auf 5°C gekühlt. Dazu tropft man 24 ml Isopentylnitrit, rührt 3 Stunden und saugt den Niederschlag ab. Der gelbe

Felstoff wird in 200 ml 5 N Schwefelsäure eingerührt. Dabei fällt das hellgelbe Produkt aus.
Ausbeute: 22 g.
Rf (Kieselgel, Fließmittel: Chloroform/Methanol/Eisessig 9:1:0,1): 0,53.

b) 6-Methylresazurin-4-carbonsäure
15,3 g 2-Methyl-4-nitrosoresorcin, 15,4 g 2,6-Dihydroxybenzosäure, 8,8 g Braunstein und 11 ml konzentrierte Schwefelsäure werden analog Beispiel 1a) umgesetzt.
Ausbeute: 28,7 g.
Rf (Kieselgel, Fließmittel: Chloroform/Methanol/Eisessig 9:1:0,1): 0,15.

c) N,O,O-Triacetyl-6-methyldihydroresorufin-4-carbonsäure
Aus 10 g 6-Methylresazurin-4-carbonsäure, 19,8 g Zinn-II-chlorid, 20 ml Acetanhydrid und 150 ml Eisessig erhält man analog Beispiel 1b) direkt die triaceylierte Leukoverbindung. Das Rohprodukt wird durch Auskochen mit Aceton gereinigt.
Ausbeute: 7,3 g.
Rf (Kieselgel, Fließmittel: Chloroform/Methanol/Eisessig 9:1:0,1): 0,51.
$^1$H-NMR ([$D_6$]-DMSO): δ = 2.10, 2.25, 2.29, 2.33 (je s, 12H); 7.00, 7.09, 7.50 und 7.74 ppm (je d, J=8.8Hz, 4H).

d) N,O,O-Triacetyl-6-methyldihydroresorufin-4-carbonsäure-N'-BOC-piperazid
Analog den Beispielen 1d) bis 1e) erhält man aus 5 g N,O,O-Triacetyl-6-methyldihydroresorufin-4-carbonsäure, 10,7 ml Oxalylchlorid und 2 g N-BOC-Piperazin 3 g Produkt.
Rf (Kieselgel, Fließmittel: Essigester): 0,57.

e) 6-Methylresorufin-4-carbonsäurepiperazid-trifluoracetat
1 g des in Beispiel 9d) beschriebenen Triacetylderivats werden analog den Beispielen 1g) bis 1h) umgesetzt.
Ausbeute: 0,43 g.

f) Kupplung von 6-Methylresorufin-4-carbonsäurepiperazid mit 2-(1-Diphenylhydantoinyl)essigsäure-N-hydroxysuccinimidester
222 mg der in Beispiel 9e) erhaltenen Verbindung werden mit 200 mg 2-(1-Diphenylhydantanoinyl)essigsäure-N-hydroxysuccinimidester umgesetzt.
Ausbeute: 250 mg.
UV/VIS (0,1 M Kaliumphosphatpuffer, pH 8.0) $\lambda_{max}$ = 584 nm.
Fluoreszenzemission: $\lambda_{max}$ = 600 nm.

Beispiel 10

Kupplung von 9-Hydroxy-5-benzo[a]phenoxazon-8-carbonsäure-piperazid mit [2-(1-Diphenylhydantoinyl)essigsäure-N-hydroxysuccinimidester

a) 9-Hydroxy-5-benzo[a]phenoxazon-8-carbonsäure-12-oxid
2,84 g 1,3-Dihydroxy-4-nitrosonaphtalin, 2,31 g 2,6-Dihydroxybenzoesäure, 1.29 g Braunstein und 1,6 ml konzentrierte Schwefelsäure werden analog Beispiel 1a) umgesetzt.
Ausbeute: 2,8 g.
Rf (Kieselgel, Fließmittel: n-Butanol/Eisessig/Wasser 4:1:1): 0,63.

b) 12-Acetyl-5,9-diacetoxybenzo[a]phenoxazon-8-carbonsäure
Analog Beispiel 9c) erhält man aus 2,4 g 9-Hydroxy-5-benzo[a]phenoxazon-8-carbonsäure-12-oxid 1,8 g der triacetylierten Dihydroverbindung.
Rf (Kieselgel, Fließmittel: Chloroform/Methanol/Eisessig 9:1:0,1): 0,31.

c) 12-Acetyl-5,9-diacetoxybenzo[a]phenoxazon-8-carbonsäure-N'-BOC-piperazid
1,6 g der in Beispiel 10b beschriebenen Triacetylverbindung werden analog Beispiel 9d) mit Oxalylchlorid und N-BOC-Piperazin umgesetzt.
Ausbeute: 1,2 g.
$^1$H-NMR ($CDCl_3$): δ = 1,49 (s, 9H); 2.12; 2.27; 2,46 (je s, 12H); 3.0—3.9 (m, 8H); 7.03 (d, J=9Hz, 1H); 7.16—7.94 ppm (m, 6H).

d) 9-Hydroxy-5-benzo[a]phenoxazon-8-carbonsäure-N'-BOC-piperazid
Analog Beispiel 1f) erhält man aus 0,93 g der Triacetylverbindung von Beispiel 10c) 0,51 g Produkt.
Rf (Kieselgel, Fließmittel: Chloroform/Methanol/Eisessig 9:1:0,1): 0,69.

e) 9-Hydroxy-5-benzo[a]phenoxazon-8-carbonsäurepiperazid-trifluoracetat
Aus 0,5 g der in Beispiel 10d) beschriebenen BOC-geschützten Verbindung erhält man analog Beispiel 1h) 0,5 g Produkt.
Rf (Kieselgel, Fließmittel: Chloroform/Methanol/Eisessig 9:1:0,1): 0,02.

f) Kopplung von 9-Hydroxy-5-benzo[a]phenoxazon-8-carbonsäurepiperazid mit 2-(1-Diphenylhydantoinyl)essigsäure-N-hydroxysuccinimidester.

Aus 50 mg Piperazid, hergestellt nach Beispiel 10e) und 150 mg 2-(1-Diphenylhydantoinyl)essigsäure-N-hydroxy succinimidester erhält man analog Beispiel 1i) 70 mg Produkt.

Rf (Kieselgel, Fließmittel: Chloroform/Methanol/Eisessig 9:1:0,1): 0,57.

UV/VIS (0.1 M Kaliumphosphatpuffer, pH 8.0) $\lambda_{max}$ = 560 nm.

Fluoreszenzemission: $\lambda_{max}$ = 615 nm.

$^1$H-NMR ([D$_6$)-DMSO): $\delta$ = 3.0—4.5 (m, 10H); 6.37 (s, 1H); 6.80 (d, J=8Hz, 1H); 7.2—7.35 (m, 10H); 7.35—8.0 (m, 3H); 8.10 (dd, J=8 und 2Hz, 1H); 8.56 (dd, J=8 und 2Hz, 1H); 9.60 ppm (s, 1H).

## Beispiel 11

8-Ethylresorufin-4-carbonsäure (1-diphenylhydantoinylmethyl carbonyl)piperazid

a) 6-Ethyl-4-nitrosoresorcin

Analog Beispiel 9a) erhält man aus 7,5 g 4-Ethylresorcin, 4,5 g Kalium-hydroxid und 8 ml Isopentylnitrit 6-Ethyl-4-nitrosoresorcin als gelben Feststoff.

Ausbeute: 7,5 g.

Rf (Kieselgel, Fließmittel: Chloroform/Methanol/Eisessig 9:1:0,1): 0,37.

b) 8-Ethylresorufin-4-carbonsäure

Analog Beispiel 1a) erhält man aus 7,4 g 6-Ethyl-4-nitrosorescorcin, 6,8 g 2,6-Dihydroxybenzosäure, 3,9 g Mangandioxid und 5 ml konz. Schwefelsäure nach Reduktion mit 8 g Zinkstaub 9,5 g Produkt.

Rf (Kieselgel, Fließmittel: Chloroform/Methanol/Eisessig 9:1:0,1): 0,05.

c) N,O,O-Triacetyl-8-ethyldihydroresorufin-4-carbonsäure N′-BOC-piperazid

Analog Beispiel 1b) erhält man aus 7,7 g 8-Ethylresorufin-4-carbonsäure, 15,4 g Zinn (II)chlorid, 30 ml Eisessig und 15,3 ml Acetanhydrid N,O,O-Triacetyl-8-ethylresorufin-4-carbonsäure, die als Rohprodukt analog Beispiel 1c) direkt zum Säurechlorid und analog Beispiel 1e) zum BOC-Piperazid weiter verarbeitet wird.

Ausbeute: 4 g.

Rf (Kieselgel, Fließmittel: Chloroform/Methanol/Eisessig 9:1:0,1): 0,86.

d) 8-Ethylresorufin-4-carbonsäure-N′-BOC-piperazid

Aus 4 g N,O,O-Triacetyl-8-ethyldihydroresorufin-4-carbonsäure N′-BOC-piperazid erhält man analog Beispiel 1f) und 1g) das entsprechende Carbonsäure-N′-BOC-piperazid.

Ausbeute: 0,5 g.

Rf (Kieselgel, Fließmittel: Chloroform/Methanol 4:1) = 0,67.

e) 8-Ethylresorufin-4-carbonsäurepiperazid Trifluoracetat

330 mg des entsprechenden BOC-Piperazids werden 1,5 Stunden in 35 ml Dichlormethan/Trifluoressigsäure 6:1 stehen gelassen. Nach Eindampfen digeriert man mit Ether, saugt ab und trocknet.

Ausbeute: 350 g.

Rf (Kieselgel, Fließmittel: Butanol/Eisessig/Wasser 4:1:1) = 0,33.

f) Umsetzung mit 2-(1-Diphenylhydantoinyl)essigsäure-N-hydroxysuccinimidester

Analog Beispiel 1i) erhält man aus 325 mg 8-Ethylresorufin-4-carbonsäurepiperazid-Trifluoracetat und 435 mg 2-(1-Diphenylhydantoinyl)essigsäure-N-hydroxysuccinimidester 120 mg Produkt.

Rf (Kieselgel, Fließmittel: Chloroform/Methanol/Eisessig 9:1:0,1) = 0,43.

$^1$H-NMR([D]$_6$-DMSO): $\delta$ = 1.15 (t, J=7.2Hz, 3H); 2.52 (q, J=7.2Hz, 2H); 3.1—4.0 (m, 8H); 4.25—4.45 (m, 2H); 6.42 (s, breit, 1H); 6.94 (d, J=9.0Hz, 1H); 7.3—7.5 (m, 11H); 7,68 (d, J=9.0Hz, 1H); 9.54 (s, 1H); 11.2 ppm (s, breit, 1H).

UV/VIS (0.1 M Kaliumphosphatpuffer pH 8.0): $\lambda_{max}$ = 575 nm.

Fluoreszenzemission: $\lambda_{max}$ = 598 nm.

## Beispiel 12

8-Chlorresorufin-4-carbonsäure-(1-diphenylhydantoinylmethyl carbonyl)piperazid

a) 8-Chlorresazurin-4-carbonsäure

Aus 17,3 g 4-Chlor-6-nitrosoresorcin (hergestellt nach Plampin und Cain, J. Med. Chem. *6,* 247 (1963)), 15,4 g 2,6-Dihydroxybenzoesäure, 8,6 g Braunstein und 10,7 ml konz. Schwefelsäure erhält man analog Beispiel 1a) 8-Chlorresazurin-4-carbonsäure.

Ausbeute: 17,1 g.

Rf (Kieselgel, Fließmittel: Butanol/Eisessig/Wasser 4:1:1) = 0,58.

b) N,O,O-Triacetyl-8-chlordihydroresorufincarbonsäure

16,3 g 8-Chlorresazurin-4-carbonsäure und 18,9 g Zinn-II-Chlorid werden 1/2 Stunde in 100 ml Eisessig/Acetanhydrid 1:1 auf 80°C erhitzt und dann auf 500 ml Eiswasser gegeben. Man rührt zwei Stunden, filtriert

vom Niederschlag und trocknet über Sicapent®. Der Feststoff wird in 500 ml Aceton aufgenommen und von ungelösten Resten filtriert. Das Filtrat dampft man ein und erhält nach Trocknen 12,3 g Produkt.

Rf (Kieselgel, Fließmittel: Chloroform/Methanol/Eisessig 9:1:0,1) = 0,39.

$^{1}$H-NMR([D]$_6$-DMSO): δ = 2.25 (s, 3H); 2.33 ("s", 6H); 7.16 (d, J=8.8Hz, 1H); 7.30 (s, 1H); 7.76 (d, J=8.8Hz, 1H); 7.90 ppm (s, 1H).

c) 8-Chlorresorufin-4-carbonsäure-N'BOC-piperazid

Analog Beispiel 1b), c) und e)-g) man aus 5 g N,O,O-Triacetyl-8-chlordihydroresorufin-4-carbonsäure 0,8 g Produkt.

Rf (Kieselgel, Fließmittel: Chloroform/Methanol/Eisessig 9:1:0,1) = 0,7.

d) 8-Chlorresorufin-4-carbonsäurepiperazid Trifluoracetat

Aus 0,8 g der BOC-geschützten Verbindung aus Beispiel 12c) erhält man 0,81 g Produkt analog Beispiel 1h).

Rf (Kieselgel, Fließmittel: Chloroform/Methanol/Eisessig 9:1:0,1) = 0,07.

e) Kopplung von 8-Chlorresorufin-4-carbonsäurepiperazid mit 2-(1-Diphenylhydantoinyl)essigsäure-N-hydroxy-succinimidester

Analog Beispiel 1i) erhält man aus 400 mg 8-Chlorresorufin-4-carbonsäurepiperazid-Trifluoracetat und 410 mg 2-(1-Diphenylhydantoinyl)-essigsäure-N-hydroxysuccinimidester das gewünschte Produkt.

Ausbeute: 150 mg.

Rf (Kieselgel, Fließmittel: Chloroform/Methanol/Eisessig 9:1:0,1 = 0,38.

UV/VIS (0.1 M Kaliumphosphatpuffer pH 8.0): $\lambda_{max}$ = 581 nm.

Fluoreszenzemission: $\lambda_{max}$ = 597 nm.

## Beispiel 13

## Kopplung von 8-Chlororesorufin-1-carbonsäure-piperazid mit Theophyllin-7-propionsäure-(2-aminoethyl)amid

a) 8-Chlororesorufin-1-carbonsäure

Man löst 8,7 g Chlor-6-nitrosoresorcin und 7,71 g 3,5-Dihydroxybenzoesäure in 200 ml Methanol, gibt bei 0°C 4,8 g Braunstein und portionsweise 5,3 ml konzentrierte Schwefelsäure zu. Man rührt 2 Stunden bei Raumtemperatur, filtriert und gibt bis zum Farbumschlag nach blau Ammoniak und 200 ml Wasser zu. Die Lösung wird filtriert, das Filtrat mit 25 ml konzentriertem Ammoniak und 20 g Zinkstaub bei Eiskühlung versetzt und danach ohne weitere Kühlung ca. 15 Minuten gerührt. Man gibt 200 mg Aktivkohle zu, filtriert, säuert auf pH 2 an und zentrifugiert das ausgefallene Resorufinderivat ab.

Ausbeute: 3,9 mg.

Rf (Kieselgel, Fließmittel: n-Butanol/Eisessig/Wasser 4:1:1): 0,88.

b) N,O,O-Triacetyl-8-chlordihydroresorufin-1-carbonsäure

Aus 3,5 g 8-Chlororesorufin-1-carbonsäure erhält man analog Beispiel 1b) 3,2 g Produkt.

Rf (Kieselgel, Fließmittel: Chloroform/Methanol/Eisessig 9:1:0,1) = 0,43.

c) 8-Chlororesorufin-1-carbonsäurepiperazid-trifluoracetat

Aus 3 g Triacetylverbindung, hergestellt in Beispiel 11b), erhält man analog den Beispielen 1c) bis 1h) 1,4 g Produkt.

Rf (Kieselgel, Fließmittel: Chloroform/Methanol/Eisessig 9:1:0,1): 0,08.

d) Kupplung von 8-Chlororesorufin-1-carbonsäurepiperazid mit Theophyllin-7-propionsäure-(2-aminoethyl)amid

Analog Beispiel 8 erhält man aus 420 mg N,O,O-Triacetyl-8-chlor-dihydroresorufin-1-carbonsäure-piperazid und 300 mg Theophyllin-7-propionsäure (2-aminoethyl)amid 190 mg Produkt.

## Beispiel 14

## Markierung von Immunoglobulin G mit N-(4-Resorufinylcarbonyl)sarcosin-N'-hydroxysuccinimidester

100 mg Human-IgG werden in 10 ml 0.1 M Kaliumphosphatpuffer pH 8.0 gelöst und mit 5 mg N-(4-Resorufinylcarbonyl)sarcosin-N'-hydroxysuccinimidester (vgl Beispiel 7e) versetzt. Man läßt 12 Stunden bei Raumtemperatur stehen und chromatographiert dann an Ultrogel ACA 202 (LKB). Das markierte Protein wird dabei vor dem freien niedermolekularen Resorufin eluiert. Der Markierungsgrad wird mittels Extinktionsmessung ermittelt. Er beträgt 3, d.h. pro Molekül IgG sind 3 Moleküle des Resorufinderivates gebunden.

## Beispiel 15

## Markierung von Immunglobulin G mit N-(4-Resorufinylcarbonyl)piperidin-4-carbonsäure-N'-hydroxysuccinimidester

a) N-(4-Resorufinylcarbonyl)piperidin-4-carbonsäure

2,0 g des Beispiel 1c) beschriebenen N,O,O-Triacetyldihydroresorufin-4-carbonsäurechlorids werden

analog Beispiel 1e) mit 0,9 g Piperidin-4-carbonsäuremethylester-Hydrochlorid umgesetzt, analog Beispiel 1f) und 1g) deacetyliert nd oxidiert und mit Natronlauge zu N-(4-Resorufinylcarbonyl)piperidin-4-carbonsäure verseift.

Ausbeute: 0,9 g.
Rf (Kieselgel RP-18, Fließmittel: Nitromethan/Ethanol 4:1) = 0,44.
UV/VIS (0.1 M Kaliumphosphatpuffer pH 8,5): $\lambda_{max}$ = 576,2 nm.

b) N-(4-Resorufinylcarbonyl)piperidin-4-carbonsäure-N'-hydroxysuccinimidester

Aus 200 mg N-(4-Resorufinylcarbonyl)piperidin-4-carbonsäure, 240 mg N-Hydroxysuccinimid und 468 mg Dicyclohexylcarbodiimid erhält man analog Beispiel 7e) 190 mg Produkt.
Rf (Kieselgel RP-18, Fließmittel: Nitromethan/Ethanol 4:1) = 0,7.
IR (KBr-Preßling): 3415 (m, breit), 1814 (w), 1773 (m), 1734 (s), 1626 (m), 1214 (m) $cm^{-1}$.

c) Markierung von Kaninchen-IgG mit N-(4-Resorufinylcarbonyl)piperidin-4-carbonsäure-N'-hydroxysuccinimidester

10 mg Kaninchen-IgG, gelöst in 1 ml 0,1 M Kaliumphosphatpuffer pH 8,5 werden mit 100 µl einer Lösung von 1,9 mg N-(4-Resorufinylcarbonyl)piperidin-4-carbonsäure-N'-hydroxysuccinimidester in 1 ml 1,4-Dioxan versetzt und zwei Stunden bei Raumtemperatur stehen gelassen. Dies entspricht einem Verhältnis von 6,4 mol Resorufin-Derivat zu 1 mol Kaninchen-IgG.

Nach Chromatographie an ACA 202 (Eluent: 0,1 M Kaliumphosphatpuffer pH 8,5) erhält man eine Proteinfraktion, die ein Absorptionsverhältnis $A_{578}/A_{280}$ = 0,97 aufweist, entsprechend einem Beladungsgrad von 3,4 mol Resorufin pro Mol IgG.

Versetzt man in einem analogen Versuch 10 mg Kaninchen-IgG mit 20 µl der Lösung des aktivierten Resorufins, erhält man bei 1,05 mol angebotenem Farbstoff pro Mol IgG einen Beladungsgrad von 0,8.

Das Absorptionsmaximum des resorufinmarkierten IgG liegt bei 578 nm. Die Lösung fluoresziert stark mit hellroter Farbe.

Setzt man eine Lösung des resorufinmarkierten IgG einen Monat dem Tageslicht aus, sinkt die Fluoreszenzintensität auf 59% des ursprünglichen Wertes, während ein analog hergestelltes mit Fluoresceinisothiocyanat markiertes IgG auf 16% und ein mit Texas Rot markiertes IgG auf 12% absinkt.

## Beispiel 16

### Diphenylhydantoin-Bestimmung in Humanserum mittels FPIA

1950 µl 0,1 M Natriumphosphatpuffer (pH 7,8) werden mit 5µl Probe[(1)], 25 µl Antikörperlösung[(2)] und 25 µl Diphenylhydantoin-Resorufinlösung[(3)] versetzt. Nach 5-minütiger Inkubation bei 37°C wird die Fluoreszenzpolarisation gemessen. (Anregungswellenlänge: 575 nm, Emissionswellenlänge 594 nm, Messgerät: Fluoreszenz-Spektrometer 650-10S, Hitachi.

1) Probe: mit bekannter Menge Diphenylhydantoin aufgestocktes humanes Spenderserum. Zur Aufstellung einer Eichkurve wird humanes Spenderserum, welches Diphenylhydantoin in Konzentrationen von

a) 2,5 µ/ml
b) 5 µ/ml
c) 10 µ/ml
d) 20 µ/ml
e) 40 µ/ml

enthält, verwendet.

2) Antikörperlösung: 450 µg Antikörper/ml 0,1 M Natriumphosphat-Puffer (pH 7,8).

Die Antikörper werden durch Immunisierung von Schafen mit Diphenylhydantoin, das an Rinderserumalbumin gebunden ist, nach üblichen Verfahren gewonnen.

Das Antiserum wurde über Ammoniumsulfatfällung und Chromatographie an DEAE-Cellulose gereinigt.

3) Diphenylhydantoinresorufinlösung ($10^{-6}$ M): Diphenylhydantoin-Resorufinkonjugate aus Beispiel 1i) in 0,1 M Natriumphosphat-Puffer (pH 7,8).

Die Meßergebnisse, die mit den Diphenylhydantoin-Lösungen 1a), 1b), 1c), 1d) und 1e) erhalten werden, sind in Abb. 1 dargestellt. Es sind dort die Diphenylhydantoinkonzentrationen der Proben (µg/ml) gegen die gemessenen Polarisationswerte (mP) aufgetragen.

Mit Hilfe einer solchen Eichkurve kann auch die Diphenylhydantoin-Konzentration in Proben mit unbekanntem Diphenylhydantoin-Gehalt bestimmt werden.

Eine vergleichbare Eichkurve erhält man auch, wenn anstelle des oben eingesetzten Diphenylhydantoinkonjugates aus Beispiel 1i) jeweils das Diphenylhydantoinkonjugat aus den Beispielen 2), 7), 8) oder 10f) verwendet wird.

Beispiel 17

Bestimmung einer Endoglycosidaseaktivität mit Resorufin-highmannose-glycopeptid.

a) Markierung des Highmannoseglycopeptids mit N-(4-Resorufinylcarbonyl)sarcosin-N'-hydroxysuccinimidester

50 mg Highmannoseglycopeptid (hergestellt nach Huang et al., Carbohydrate Res., *13*, 127—137 (1970)) werden mit 10 ml 0,1 M Kaliumphosphatpuffer pH 8,0 versetzt. Die Lösung wird auf einen pH von 8,0 nachgestellt. 25 mg N-(4-Resorufinylcarbonyl)sarcosin-N'-hydroxysuccinimidester gelöst in 3 ml Dioxan werden zugegeben, nach 1 Stunde nochmals dieselbe Menge Farbstoff-N-hydroxysuccinimidester in 3 ml Dioxan. Man rührt 14 Stunden bei Raumtemperatur, dampft dann im Vakuum das Dioxan ab und verdünnt mit Wasser auf 70 ml, danach mit Puffer A (= 0,02 M Tris-HCl, 2 mM Magnesiumchlorid, 2 mM Mangan-II-chlorid, 2 mM Calciumchlorid, pH 7,2) auf 140 ml. Der pH wird mit wässrigen Ammoniak auf 7,2 nachgestellt. Dabei gebildeter Niederschlag wird abzentrifugiert. Der Überstand wird auf eine Con A-Sepharosesäule (1 × 15 cm) gegeben. Mit Puffer A wird freier Farbstoff ausgewaschen. Sobald der Durchlauf nicht mehr rot ist, eluiert man eine 1. Fraktion Resorufin-Highmannoseglycopeptid mit 2% Methylmannosid in Puffer A als Eluent (ca. 100 ml). Danach eluiert man eine 2. Fraktion mit 2 %igem wässrigen Methylmannosid. Beide Fraktionen werden gegen Wasser dialysiert und lyophilisiert, beide Fraktionen eignen sich für die unter (b) beschriebene Bestimmung der Endoglycosidaseaktivität.

b) Bestimmung der Endoglycosidaseaktivität

Resorufin-Highmannoseglycopeptid wird in einem geeigneten Puffer mit Endoglycosidase inkubert, z.B. Endoglycosidase H und Citrat-Puffer pH 5,5 (= Probe 1). Parallel dazu wird eine Probe mitgeführt, die keine Endoglycosidase enthält, sonst aber identisch ist (= Probe 2). Nach Inkubation werden beide Proben mit Con A-Sepharose versetzt und geschüttelt, um Resorufin-Highmannoseglycopeptid zu binden. Resorufinmarkiertes Peptid, bei dem durch die Enzymaktivität der Zuckerteil abgespalten worden ist, binden nicht. Nach 15 Minuten wird die Con A-Sepharose abzentrifugiert, der Überstand auf pH 7,5 gestellt und die Fluoreszenz gemessen (Anregung z.B. 550 nm, Emission $\lambda_{max}$ = 595 nm). Die Differenz zwischen Probe 1 und dem Leerwert (= Probe 2) gibt die Menge an gespaltenem Resorufin-Highmannoseglycopeptid an und ist damit ein Maß für die Enzymaktivität.

**Patentansprüche**

1. Resorufin-Derivate der allgemeinen Formeln Ia und Ib

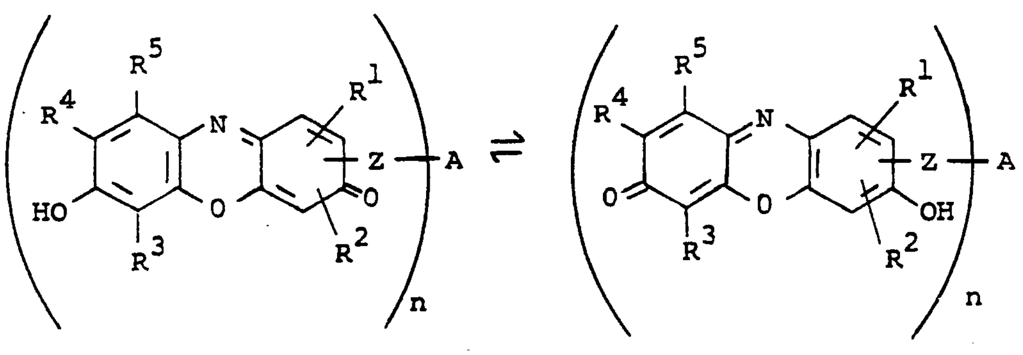

(Ia) (Ib)

in denen

$R^1$, $R^2$, $R^3$, $R^4$ und $R^5$, die jeweils gleich oder verschieden sein können, Wasserstoff, Halogen, eine Carboxy-, Carboxamido-, Niederalkoxycarbonyl-, Cyano- oder Nitrogruppe, einen Niederalkyl- oder Niederalkoxy-Rest, die jeweils durch Carboxy-, Carboxamido-, Niederalkoxycarbonyl-, Cyano- oder Nitrogruppen substituiert sein können, wobei $R^4$ und $R^5$ zusammen einen anellierten Benzol- oder Naphthalinring vorstellen können der unsubstituiert ist oder einen oder mehrere Substituenten, ausgewählt aus der Gruppe $SO_3H$, $CO_2H$ oder $C_1$—$C_5$-Alkoxy, trägt,

Z ein Brückenglied

A den Rest eines Liganden und

n eine ganze Zahl von bis 200

bedeuten, wobei die Niederalkyl- bzw. Niederalkoxygruppen Kohlenwasserstoffketten mit 1 bis 5 Kohlenstoffatomen enthalten.

2. Resorufin-Derivate gemäß Anspruch 1, dadurch gekennzeichnet, daß der Rest A ein Hapten, Antigen, Antikörper, Substrat oder Träger bedeutet.

3. Verfahren zur Herstellung von Resorufin-Derivaten der allgemeinen Formeln Ia und Ib

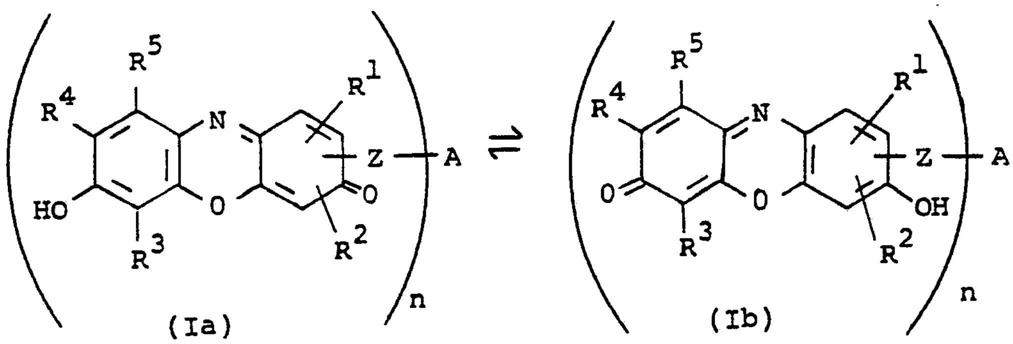

(Ia) (Ib)

in denen

$R^1$, $R^2$, $R^3$, $R^4$ und $R^5$, die jeweils gleich oder verschieden sein können, Wasserstoff, Halogen, eine Carboxy-, Carboxamido-, Niederalkoxycarbonyl-, Cyano- oder Nitrogruppe, einen Niederalkyl- oder Niederalkoxy-Rest, die jeweils durch Carboxy-, Carboxamido-, Niederalkoxycarbonyl-, Cyano- oder Nitrogruppen substituiert sein können, wobei $R^4$ und $R^5$ zusammen einen anellierten Benzol- oder Naphthalinring vorstellen können der unsbustituiert ist oder einen oder mehrere Substituenten, ausgewählt aus der Gruppe $SO_3H$, $CO_2H$ oder $C_1$—$C_5$-Alkoxy, trägt,

Z ein Brückenglied

A den Rest eines Liganden und

n eine ganze Zahl von bis 200

bedeuten, wobei die Niederalkyl- bzw. Niederalkoxygruppen Kohlenwasserstoffketten mit 1 bis 5 Kohlenstoffatomen enthalten, dadurch gekennzeichnet, daß man in an sich bekannter Weise Verbindungen der allgemeinen Formeln IIa und IIb,

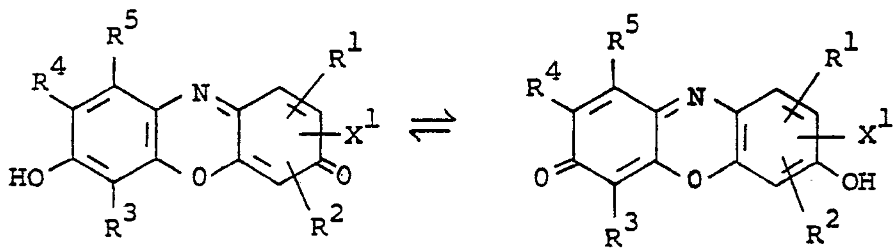

(IIa) (IIb)

in denen $R^1$, $R^2$, $R^3$, $R^4$ und $R^5$ die in den allgemeinen Formeln Ia und Ib angegebenen Bedeutungen haben und $X^1$ eine reaktive Gruppe vorstellt,

a) mit einem Liganden der allgemeinen Formel

$$X^2—A \qquad (III)$$

in der

$X^2$ eine reaktive Gruppe und

A den Rest des Liganden vorstellt,

oder

b; mit einer bifunktionellen Verbindung der allgemeinen Formel

$$X^3—M—X^4 \qquad (IV)$$

in der

$X^3$ und $X^4$ reaktive Gruppen und

M den Rest der bifunktionellen Verbindung bedeuten,

und einem Liganden der allgemeinen Formel

$$X^2—A \qquad (III)$$

in der

$X^2$ und A die oben angegebene Bedeutung haben,

umsetzt, wobei gegebenenfalls bei einzelnen Verfahrensschritten Schutzgruppen eingeführt und nachträglich wieder abgespalten sowie einzelne reaktive Gruppen $X^1$, $X^2$, $X^3$ und $X^4$ in andere reaktive Gruppen überführt werden können.

4. Verfahren gemäß Anspruch 3, dadurch gekennzeichnet, daß als bifunktionelle Verbindungen $X^3$—M—$X^4$ ein Diamin, eine Dicarbonsäure oder ein Derivat hiervon, ein Dialdehyd oder eine Aminocarbonsäure verwendet wird.

5. Verfahren gemäß Anspruch 4, dadurch gekennzeichnet, daß als bifunktionelle Verbindung Piperazin, 1,2-Ethylendiamin, Glycin, Sarcosin, β-Alanin oder Piperidin-4-carbonsäure verwendet wird.

6. Verwendung der Resorufin-Derivate gemäß einem der Ansprüche 1 oder 2 analytischen Verfahren, bei denen eine Fluoreszenzeigenschaft der Verbindungen der allgemeinen Formel Ia/Ib bzw. verfahrensbedingt entstandener Umwandlungsprodukte gemessen wird.

7. Resorufin-Derivate der allgemeinen Formel IIa und IIb

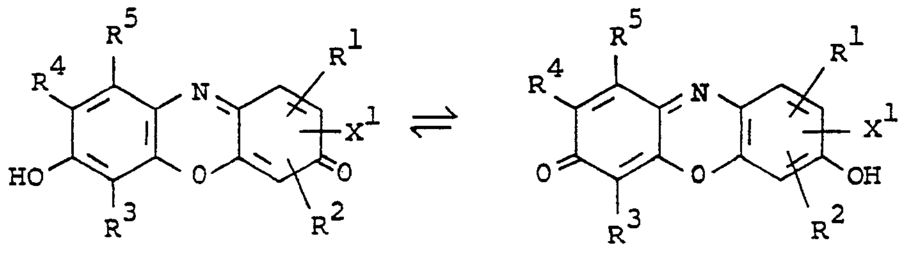

(IIa) (IIb)

in denen $R^1$, $R^2$, $R^3$, $R^4$ und $R^5$ die in den allgemeinen Formeln Ia und Ib angegebenen Bedeutungen haben und $X^1$ eine reaktive Gruppe vorstellt.

8. Verfahren zur Herstellung von Resorufin-Derivaten der allgemeinen Formel IIa und IIb

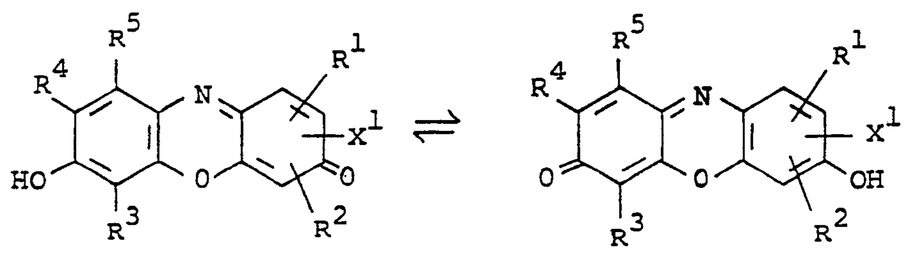

(IIa) (IIb)

in denen $R^1$, $R^2$, $R^3$, $R^4$ und $R^5$ die in den allgemeinen Formeln Ia und Ib angegebenen Bedeutungen haben und $X^1$ eine reaktive Gruppe vorstellt, dadurch gekennzeichnet daß Nitrosoresorcin-Derivate der allgemeinen Formel V

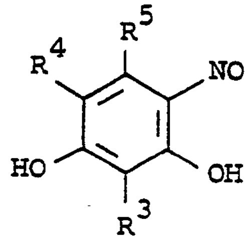

(V)

in der $R^3$, $R^4$ und $R^5$ die in den allgemeinen Formeln Ia und Ib angegebene Bedeutung besitzen mit Resorcin-Derivaten der allgemeinen Formel VI

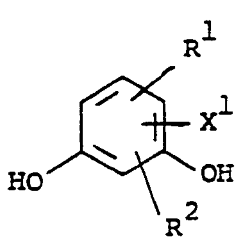

(VI)

in der $R^1$ und $R^2$ die in den allgemeinen Formeln Ia und Ib angegebene Bedeutung besitzen und $x^1$ eine reaktive Gruppe vorstellt in Gegenwart eines Oxidationsmittels bei niedrigen Temperaturen umgesetzt und dann zunächst entstehende Resazurin-Derivate reduziert werden.

9. Resorufin-Derivate der allgemeinen Formel VIIIa und VIIIb

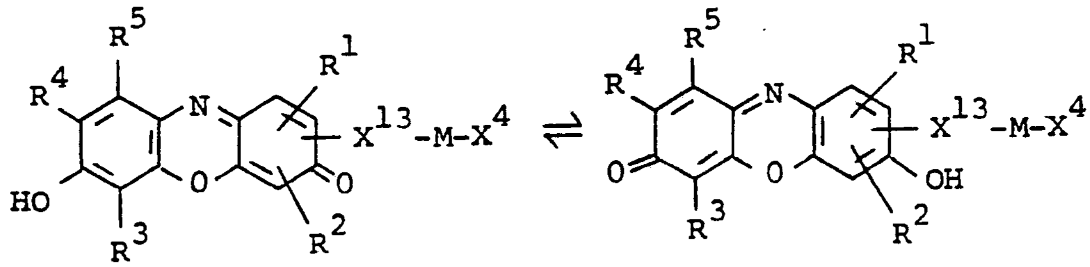

(VIIIa) (VIIIb)

in denen

$R^1$, $R^2$, $R^3$, $R^4$ und $R^5$ die in den allgemeinen Formeln Ia und Ib und

M und $X^4$ die in der allgemeinen Formel IV angegebenen Bedeutungen haben und

$X^{13}$ dine durch Reaktion von $X^1$ und $X^3$ entstehende funktionelle Gruppe vorstellen.

10. Verfahren zur Herstellung von Resorufin-Derivaten der allgemeinen Formel VIIIa und VIIIb

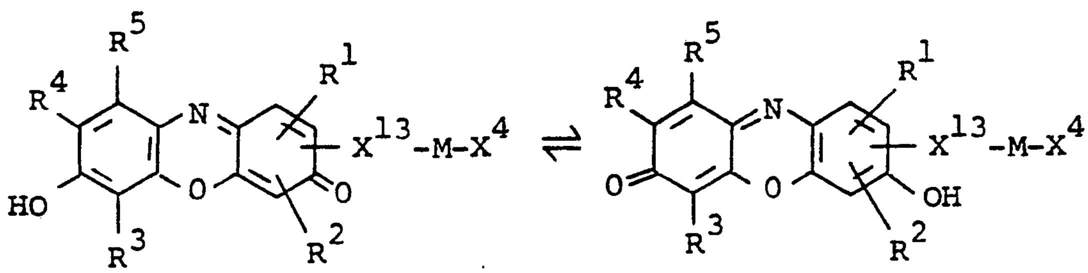

(VIIIa) (VIIIb)

in denen

$R^1$, $R^2$, $R^3$, $R^4$ und $R^5$ die in den allgemeinen Formeln Ia und Ib und

M und $X^4$ die in der allgemeinen Formel IV angegebenen Bedeutungen haben und

$X^{13}$ eine durch Reaktion von $X^1$ und $X^3$ entstehende funktionelle Gruppe vorstellen, dadurch gekennzeichnet, daß Resorufinderivate der allgemeinen Formeln IIa und IIb

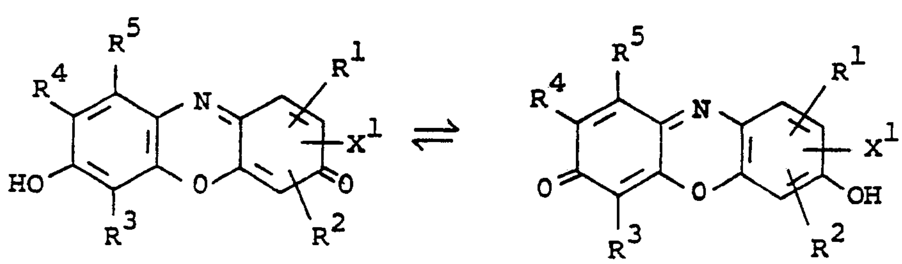

(IIa) (IIb)

in denen

$R^1$, $R^2$, $R^3$, $R^4$ und $R^5$ die in den allgemeinen Formeln Ia und Ib angegebenen Bedeutungen haben und

$X^1$ eine reaktive Gruppe vorstellt mit bifunktionellen Verbindungen der allgemeinen Formel IV

$$X^3\text{—}M\text{—}X^4 \qquad (IV)$$

in der

$X^3$ und $X^4$ reaktive Gruppen und

M den Rest einer bifunktionellen Verbindung bedeuten umgesetzt werden.

11. Verwendung der Resorufin-Derivate gemäß Anspruch 7 oder 9 zur Herstellung von Verbindungen gemäß Anspruch 1.

**Revendications**

1. Dérivés de résorufine de formules générales Ia et Ib

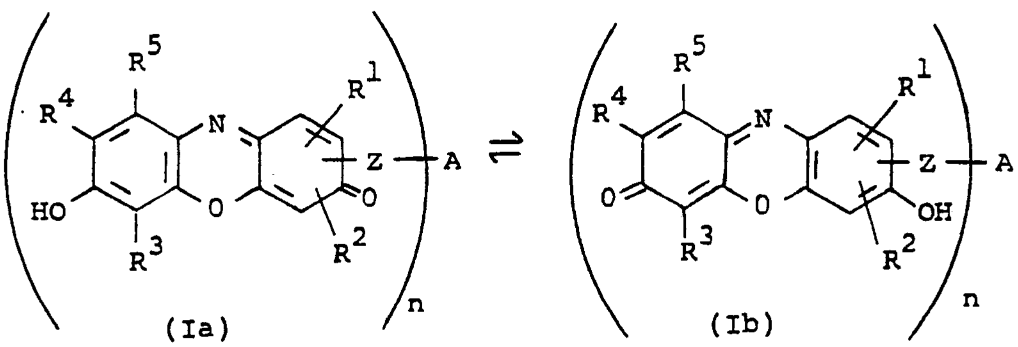

où

$R^1$, $R^2$, $R^3$, $R^4$ et $R^5$, qui peuvent être identiques ou différents, représentent un atome d'hydrogène, un atome d'halogène, un groupe carboxy, carboxyamido, alcoxycarbonyle inférieur, cyano ou nitro, un groupe alkyle inférieur ou un groupe alcoxy inférieur, qui peuvent être chacun substitués par un groupe carboxy, carboxyamido, alcoxycarbonyle inférieur, cyano ou nitro, $R^4$ et $R^5$ pouvant représenter conjointement un noyau benzénique ou naphtalénique cyclisé qui n'est pas substitué ou qui est substitué par un ou plusieurs substituants choisis dans l'enzemble formé par les groupes $SO_3H$, $CO_2H$ ou alcoxy en $C_1$—$C_5$,

Z représente un pont

A représente le reste d'un ligand et

n est un nombre intier de 1 à 200,

les groupes alkyle inférieur et alcoxy inférieur comprenant des chaînes hydrocarbonées avec 1 à 5 atomes de carbone.

2. Dérivés de résorufine selon la revendication 1, caractérisés en ce que le groupe A signifie un haptène, un antigène, un anticorps, un substrat ou un support.

3. Procédé pour la préparation de dérivés de résorufine de formules générales Ia et Ib

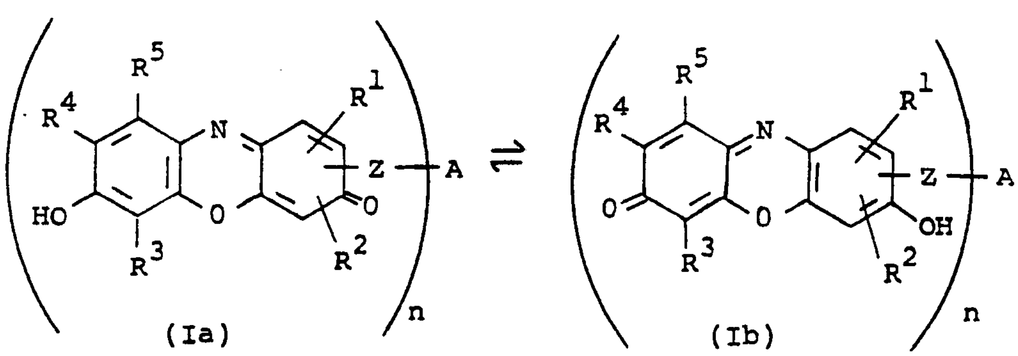

où

$R^1$, $R^2$, $R^3$, $R^4$ et $R^5$, qui peuvent être identiques ou différents, représentent un atome d'hydrogène, un atome d'halogène, un groupe carboxy, carboxyamido, alcoxycarbonyle inférieur, cyano ou nitro, un groupe alkyle inférieur ou un groupe alcoxy inférieur, qui peuvent être chacun substitués par un groupe carboxy, carboxyamido, alcoxycarbonyle inférieur, cyano ou nitro, $R^4$ et $R^5$ pouvant représenter conjointement un noyau benzénique ou naphtalénique cyclisé qui n'est pas substitué ou qui est substitué par un ou plusieurs substituants choisis dans l'enzemble formé par les groupes $SO_3H$, $CO_2H$ ou alcoxy en $C_1$—$C_5$,

Z représente un pont

A représente le reste d'un ligand et

n est un nombre intier de 1 à 200,

les groupes alkyle inférieur et alcoxy inférieur comprenant des chaînes hydrocarbonées avec 1 à 5 atomes de carbone, caractérisé en ce que, de manière connue en soi, on fait réagir des composés de formules générales IIa et IIb,

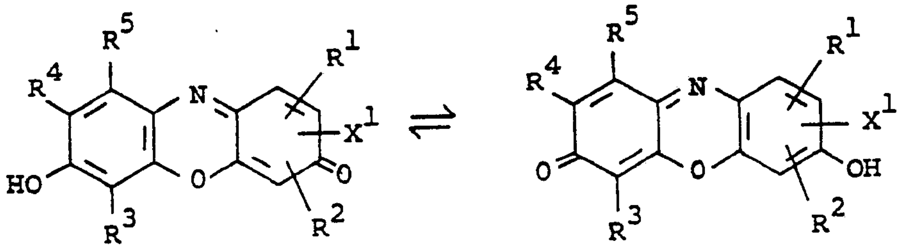

(IIa) (IIb)

où

$R^1$, $R^2$, $R^3$, $R^4$ et $R^5$ ont les significations mentionnées dans les formules générales Ia et Ib et $X^1$ représente un groupe réactif

a) avec ligand de formule générale

$$X^2—A \qquad (III)$$

où

$X^2$ représente un groupe réactif et

A représente le rste du ligand,

ou

b) avec un composé bifonctionnel de formule générale

$$X^3—M—X^4 \qquad (IV)$$

où

$X^3$ et $X^4$ représentent des groupes réactifs et

M représente le reste du composé bifonctionnel,

et avec un ligand de formule générale

$$X^2—A \qquad (III)$$

où

$X^2$ et A ont les significations mentionnées ci-dessus,

des groupes de protection pouvant être introduits dans les étapes individuelles et ensuite, séparés de nouveau, et les groupes réactifs individuels $X^1$, $X^2$, $X^3$ et $X^4$ peuvent être transformés en d'autres groupes réactifs.

4. Procédé selon la revendication 3, caractérisé en ce que, comme composés bifonctionnels $X^3—M—X^4$, on utilise un diamine, un acide dicarboxylique ou un de ses dérivés, un dialdéhyde ou un acide aminocarboxylique.

5. Procédé selon la revendication 4, caractérisé en ce que comme composé bifonctionnel, on utilise la pipérazine, la 1,2-éthylènediamine, la glycine, la sarcosine, la ω-alanine ou l'acide pipéridine-4-carboxylique.

6. Utilisation des dérivés de résorufine selon l'une des revendications 1 ou 2 dans des procédés analytiques, dans lesquels on mesure la propriété de fluorescence du composé de formule générale Ia/Ib, ou celle du produit formé suivant le procédé.

7. Dérivés de résorufine de formules générales IIa et IIb

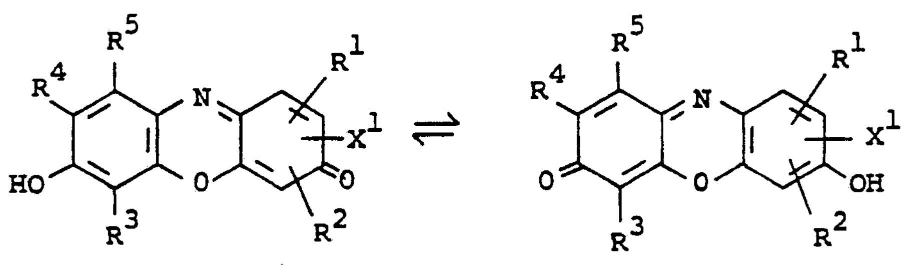

(IIa) (IIb)

où

$R^1$, $R^2$, $R^3$, $R^4$ et $R^5$ ont les significations mentionnées dans les formules générales Ia et Ib et $X^1$ représente un groupe réactif.

8. Procédé pour la préparation de dérivés de résorufine de formules générales IIa et IIb

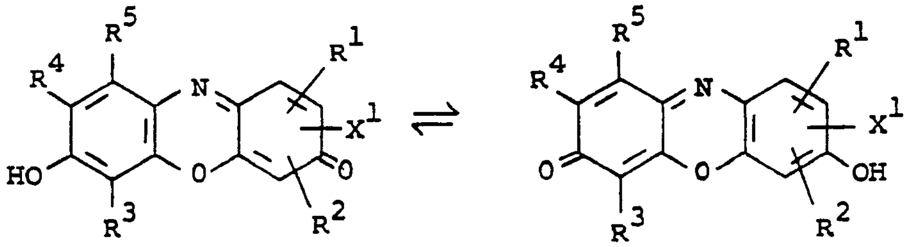

(IIa) (IIb)

où

$R^1$, $R^2$, $R^3$, $R^4$ et $R^5$ ont les significations mentionnées dans les formules générales Ia et Ib et $X^1$ représente un groupe réactif, caractérisé en ce que l'on fait réagir des dérivés de nitrosorésorcine de formule générale V

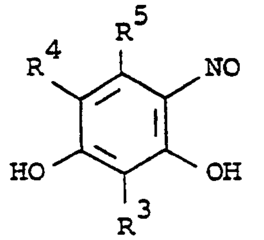

(V)

où

$R^3$, $R^4$ et $R^5$ ont les significations mentionnées dans les formules générales Ia et Ib, avec des dérivés de résorcine de formule générale VI

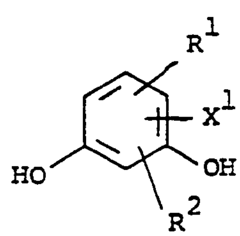

(VI)

où

$R^1$ et $R^2$ ont les significations mentionnées dans les formules générales Ia et Ib et $X^1$ représente un groupe réactif, en présence d'un agent oxydant, à basse température, et ensuite, on réduit des dérivés de résazurine formés en premier.

9. Dérivés de résorufine de formules générales VIIIa et VIIIb

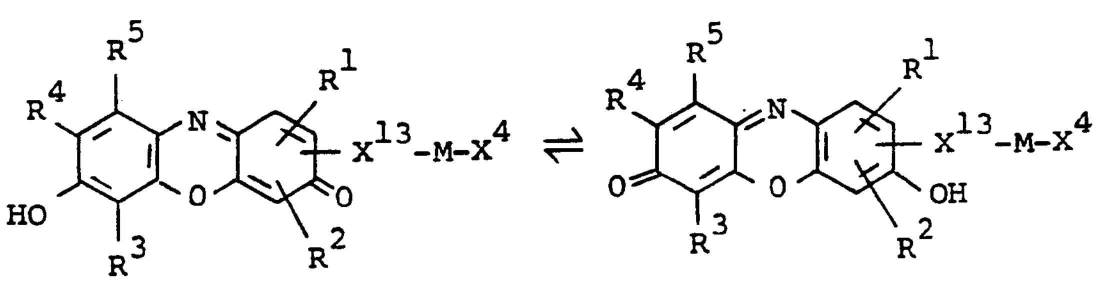

(VIIIa) (VIIIb)

où

$R^1$, $R^2$, $R^3$, $R^4$ et $R^5$ ont les significations mentionnées dans les formules générales Ia et Ib et
M et $X^4$ ont les significations mentionnées dans la formule générale IV et
$X^{13}$ représente un groupe fonctionnel formé par la réaction de $X^1$ et $X^3$.

10. Procédé pour la préparation de dérivés de résorufine de formules générales VIIIa et VIIIb

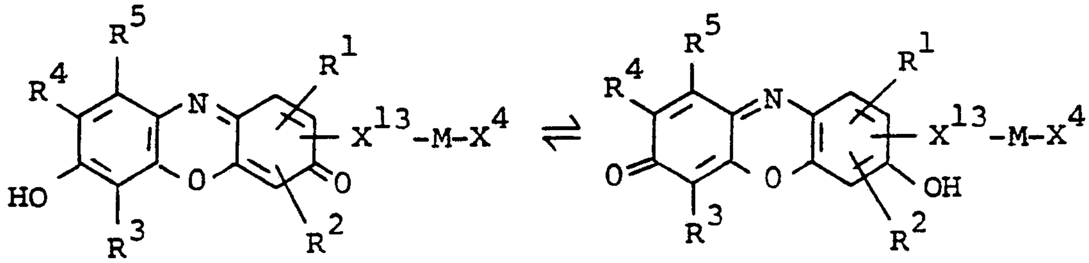

(VIIIa) (VIIIb)

où

$R^1$, $R^2$, $R^3$, $R^4$ et $R^5$ ont les significations mentionnées dans les formules générales Ia et Ib et
M et $X^4$ ont les significations mentionnées dans la formule générale IV et
$X^{13}$ représente un groupe fonctionnel formé par la réaction de $X^1$ et $X^3$,

caractérisé en ce que l'on fait réagir des dérivés de résorufine de formules générales IIa et IIb

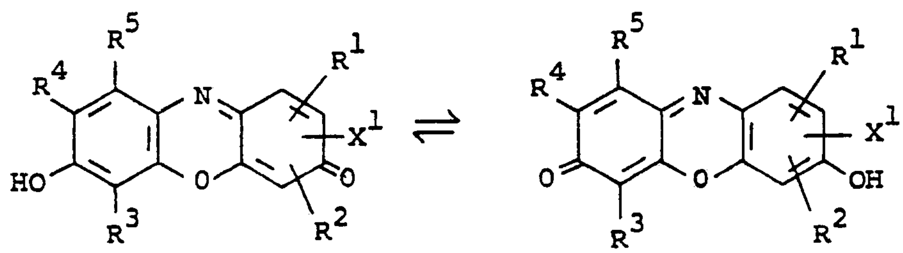

(IIa) (IIb)

où

$R^1$, $R^2$, $R^3$, $R^4$ et $R^5$ ont les significations mentionnées dans les formules générales Ia et Ib et $X^1$ représente un groupe réactif, avec des composés bifonctionnels de formule générale IV

$$X^3\text{—}M\text{—}X^4 \qquad (IV)$$

où

$X^3$ et $X^4$ représentent des groups réactifs et
M représente le reste d'un composé bifonctionnel.

11. Utilisation des dérivés de résorufine selon la revendication 7 ou 9, pour la préparation de composés selon la revendication 1.

## Claims

1. Resorufin derivatives of the general formulae Ia and Ib

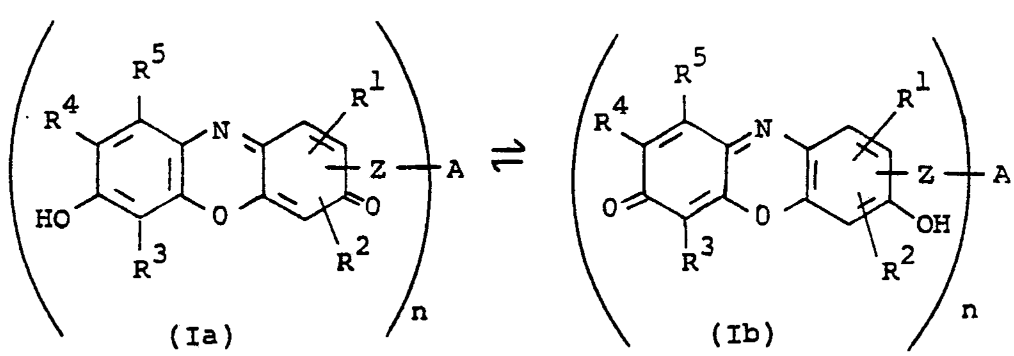

(Ia) (Ib)

in which $R^1$, $R^2$, $R^3$, $R^4$ and $R^5$, which in each case can be the same or different, signify hydrogen, halogen, a carboxyl, carboxamido, lower alkoxycarbonyl, cyano or nitro group, a lower alkyl or lower alkoxy radical, which in each case can be substituted by carboxyl, carboxamido, lower alkoxycarbonyl, cyano or nitro groups, whereby $R^4$ and $R^5$ can together represent an anellated benzene or naphthalene ring which is

unsubstituted or carries one or more constituents selected from the group $SO_3H$, $CO_2H$ or $C_1$—$C_5$-alkoxy, Z a bridge member, A the residue of a ligand and *n* a whole number of 1 to 200, whereby the lower alkyl and lower alkoxy groups contain hydrocarbon chains with 1 to 5 carbon atoms.

2. Resorufin derivatives according to claim 1, characterised in that the residue A signifies a hapten, antigen, antibody, substrate or carrier.

3. Process for the preparation of resorufin derivatives of the general formulae Ia and Ib

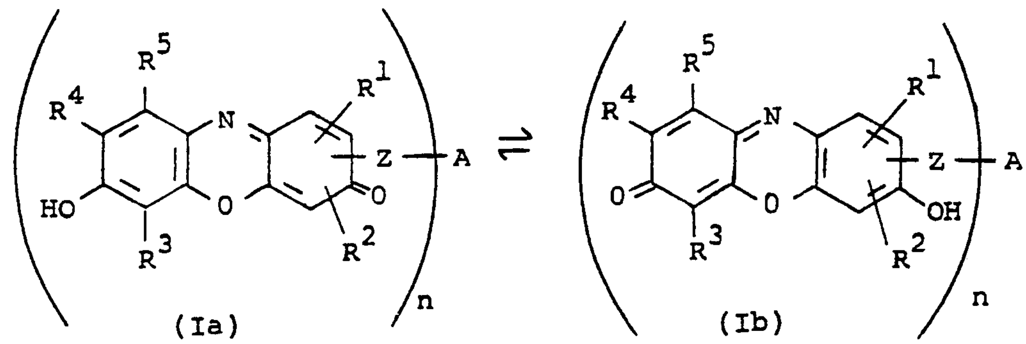

(Ia) (Ib)

in which $R^1$, $R^2$, $R^3$, $R^4$ and $R^5$, which in each case can be the same or different, signify hydrogen, halogen, a carboxyl, carboxamido, lower alkoxycarbonyl, cyano or nitro group, a lower alkyl or lower alkoxy radical, which in each case can be substituted by carboxyl, carboxamido, lower alkoxycarbonyl, cyano or nitro groups, whereby $R^4$ and $R^5$ can together represent an anellated benzene or naphthalene ring which is unsubstituted or carries one or more substituents selected from the group $SO_3H$, $CO_2H$ or $C_1$—$C_5$-alkoxy, Z is a bridge member, A the residue of a ligand and *n* a whole number of 1 to 200, whereby the lower alkyl and lower alkoxy groups contain hydrocarbon chains with 1 to 5 carbon atoms, characterised in that, in per se known way, one reacts a compound of the general formulae IIa and IIb

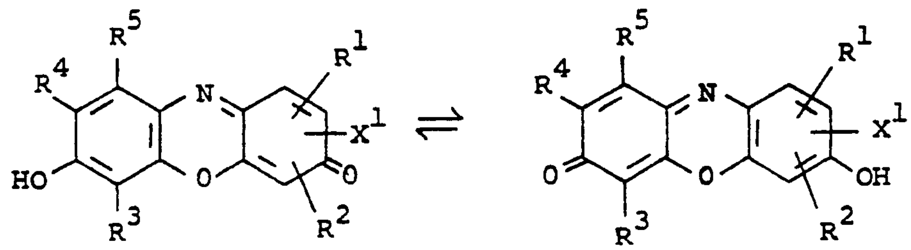

(IIa) (IIb)

in which $R^1$, $R^2$, $R^3$, $R^4$ and $R^5$ have the meanings given in the general formulae Ia and Ib and $X^1$ represents a reactive group,

a) with a ligand of the general formula:—

$$X^2—A \qquad (III)$$

in which $X^2$ represents a reactive group and A the residue of the ligand; or

b) with a bifunctional compound of the general formula:—

$$X^3—M—X_4 \qquad (IV)$$

in which $X^3$ and $X^4$ signify reactive groups and M the residue of the bifunctional compound, and a ligand of the general formula:—

$$X^2—A \qquad (III)$$

in which $X^2$ and A have the above-given meaning, whereby, in individual process steps, protective groups can be introduced and subsequently again split off, as well as individual reactive groups $X^1$, $X^2$, $X^3$ and $X^4$ converted into other reactive groups.

4. Process according to claim 1, characterised in that, as bifunctional compounds $X^3$—M—$X^4$, a diamine, a dicarboxylic acid or a derivative thereof, a dialdehyde or an aminocarboxylic acid is used.

5. Process according to claim 4, characterised in that, as bifunctional compounds, piperazine, 1,2-ethylenediamine, glycine, sarcosine, β-alanine or piperidine-4-carboxylic acid is used.

6. Use of the resorufin derivatives according to one of claims 1 or 2 in which a fluorescent property of the compounds of general formula Ia/Ib or conversion products arising as a result of the process is measured.

7. Resorufin derivatives of the general formulae IIa and IIb

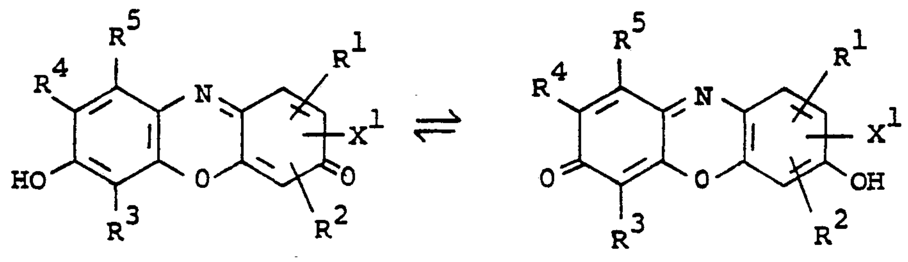

(IIa) (IIb)

in which $R^1$, $R^2$, $R^3$, $R^4$ and $R^5$ have the meanings given in the general formulae Ia and Ib and $X^1$ represents a reactive group.

8. Process for the preparation of resorufin derivatives of the general formulae IIa and IIb

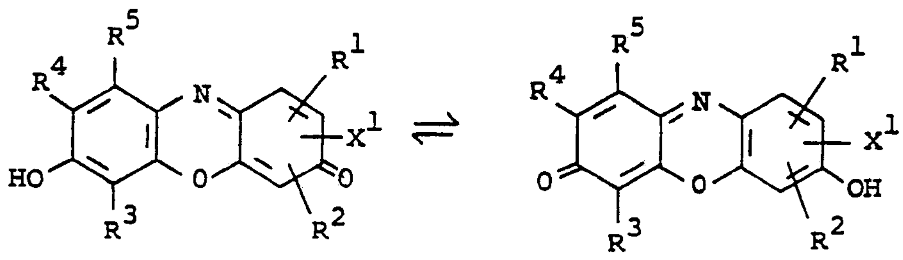

(IIa) (IIb)

in which $R^1$, $R^2$, $R^3$, $R^4$ and $R^5$ have the meanings given in the general formulae Ia and Ib and $X^1$ represents a reactive group, characterised in that nitrosoresorcinol derivatives of the general formula V

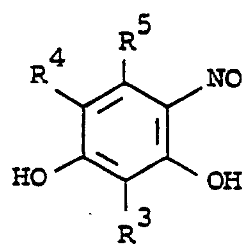

(V)

in which $R^3$, $R^4$ and $R^5$ possess the meaning given in the general formulae Ia and Ib, are reacted with resorcinol derivatives of the general formula VI

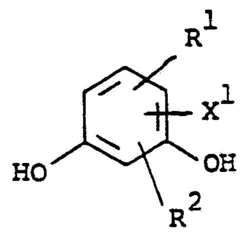

(VI)

in which $R^1$ and $R^2$ possess the meaning given in the general formulae Ia and Ib and $X^1$ represents a reactive group, in the presence of an oxidation agent at low temperatures and then initially formed resazurine derivatives are reduced.

9. Resorufin derivatives of the general formulae VIIIa aand VIIIb

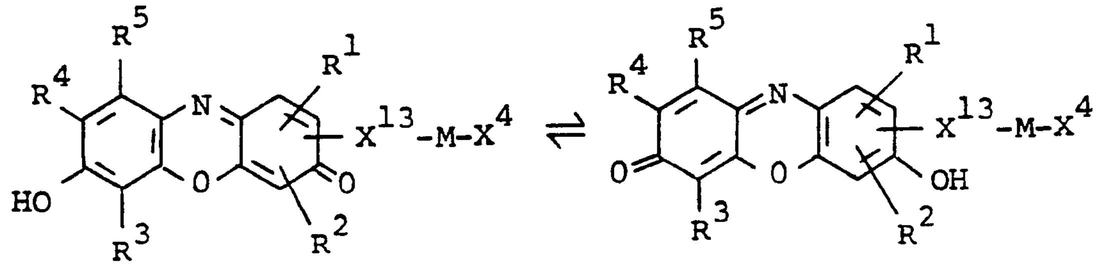

(VIIIa) (VIIIb)

in which $R^1$, $R^2$, $R^3$, $R^4$ and $R^5$ have the meanings given in the general formulae Ia and Ib and M and $X^4$ the meanings given in the general formula IV and $X^{13}$ represents a functional group resulting by reaction of $X^1$ and $X^3$.

10. Process for the preparation of resorufin derivatives of the general formulae VIIIa and VIIIb

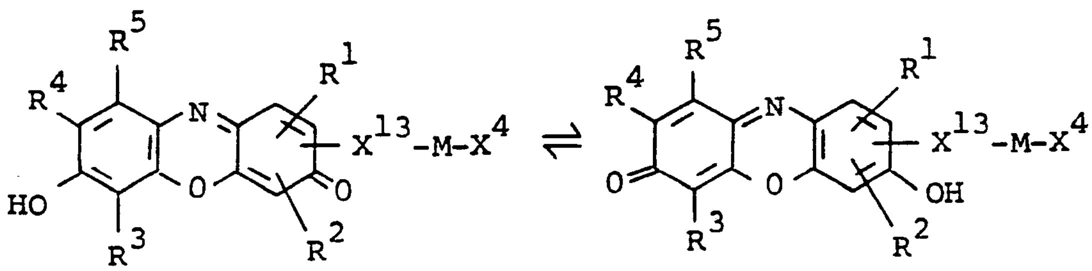

(VIIIa) (VIIIb)

in which $R^1$, $R^2$, $R^3$, $R^4$ and $R^5$ have the meanings given in the general formulae Ia and Ib and M and $X^4$ the meanings given in the general formula IV and $X^{13}$ represents a functional group resulting by reaction of $X^1$ and $X^3$, characterised in that resorufin derivatives of the general formulae IIa and IIb

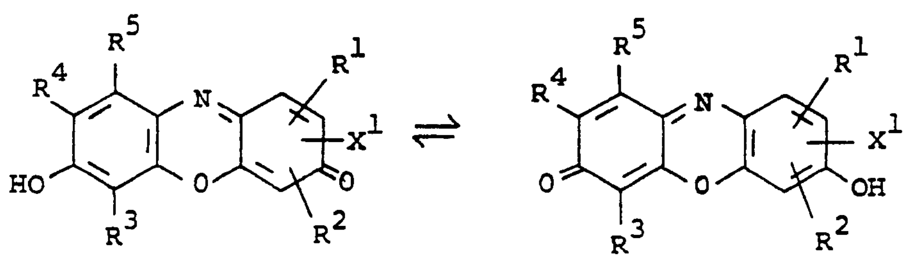

(IIa) (IIb)

in which $R^1$, $R^2$, $R^3$, $R^4$ and $R^5$ have the meanings given in the general formulae Ia and Ib and M and $X^1$ represents a reactive group, are reacted with bifunctional compounds of the general formula IV

$$X^3—M—X^4 \qquad (IV)$$

in which $X^3$ and $X^4$ signify reactive groups and M the residue of a bifunctional compound.

11. Use of the resorufin derivatives according to claim 7 or 9 for the preparation of compounds according to claim 1.

Abbildung 1

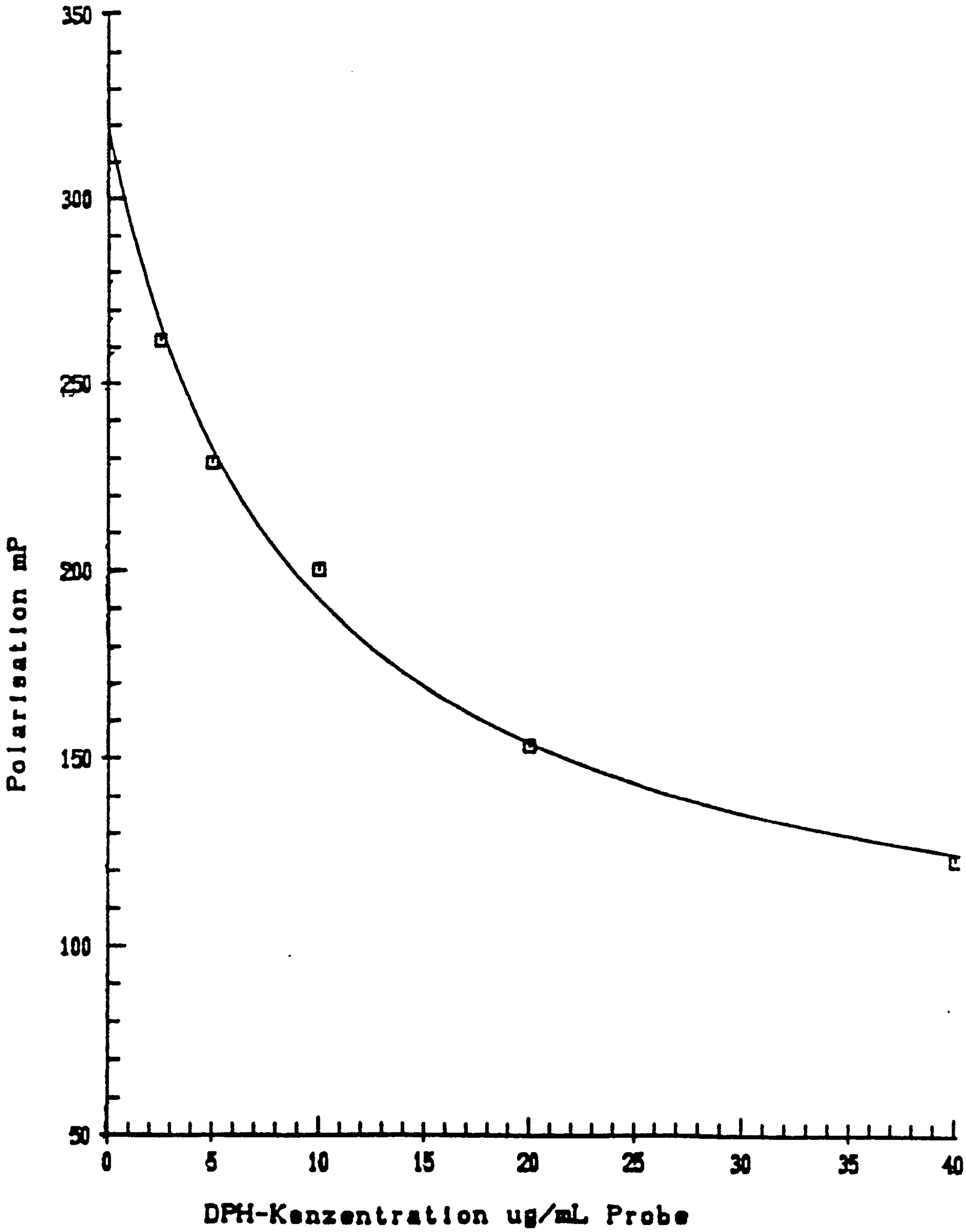
350
300
250
200
150
100
50
0
5
10
15
20
25
30
35
40
Polarisation mP
DPH-Konzentration ug/mL Probe